(19) Europäisches Patentamt
European Patent Office
Office européen des brevets

(11) EP 0 443 449 B1

(12) EUROPÄISCHE PATENTSCHRIFT

(45) Veröffentlichungstag und Bekanntmachung des Hinweises auf die Patenterteilung:
**21.05.1997 Patentblatt 1997/21**

(51) Int Cl.[6]: **C07D 305/12**, C07D 309/38, C07D 309/32, C07D 309/30

(21) Anmeldenummer: **91102150.9**

(22) Anmeldetag: **15.02.1991**

(54) **Verfahren zur Herstellung von Oxetanonen**

Process for the preparation of oxetanones

Procédé de préparation d'oxétanones

(84) Benannte Vertragsstaaten:
**AT BE CH DE DK ES FR GB GR IT LI LU NL SE**

(30) Priorität: **23.02.1990 CH 589/90**
**12.12.1990 CH 3925/90**

(43) Veröffentlichungstag der Anmeldung:
**28.08.1991 Patentblatt 1991/35**

(73) Patentinhaber: **F. HOFFMANN-LA ROCHE AG**
**4002 Basel (CH)**

(72) Erfinder:
- **Karpf, Martin, Dr.**
  **CH-4153 Reinach (CH)**
- **Zutter, Ulrich, Dr.**
  **CH-4051 Basel (CH)**

(74) Vertreter: **Lederer, Franz, Dr. et al**
**Lederer, Keller & Riederer**
**Patentanwälte**
**Prinzregentenstrasse 16**
**80538 München (DE)**

(56) Entgegenhaltungen:
**EP-A- 0 189 577** **DE-A- 2 400 429**

- **HELVETICA CHIMICA ACTA, Band 70, Nr. 5, 1987, Basel (CH); P. BARBIER et al., Seiten 1412-1418**
- **HELVETICA CHIMICA ACTA, Band 70, No. 1, 1987, Basel (CH); P. BARBIER et al., Seiten 196-202**
- **CHEMICAL ABSTRACTS, Band 110, 1989, Columbus, OH (US); J. GARNERO et al., Seite 686, Nr. 134949q**
- **JOURNAL OF ORGANIC CHEMISTRY, Band 53, Nr. 6, 1988, American Chemical Society, Washington, DC (US); P. BARBIER et al., Seiten 1218-1221**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 443 449 B1

Printed by Jouve, 75001 PARIS (FR)

## Beschreibung

Die vorliegende Erfindung betrifft ein neues Verfahren zur Herstellung von Oxetanonen der Formel

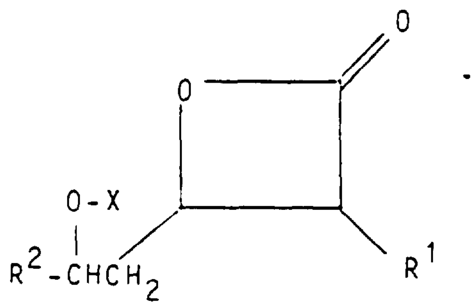

I

worin

| | |
|---|---|
| $R^1$ und $R^2$ | in einer anderen als der α- oder β-Stellung gegebenenfalls durch ein O-Atom unterbrochenes Alkyl mit bis zu 17 C-Atomen; oder gegebenenfalls durch 1 bis 3 $C_{1-6}$-Alkyl- oder $C_{1-6}$-Alkoxygruppen ringsubstituiertes Benzyl, |
| X | Wasserstoff oder eine Gruppe der Formel $(R^3,R^4)NCH(R^5)(CH_2)_n$-CO- |
| $R^3$ | Wasserstoff, $C_{1-3}$-Alkyl oder $C_{1-3}$-Alkanoyl, |
| $R^4$ | Wasserstoff $C_{1-3}$-Alkyl, und |
| $R^5$ | Wasserstoff, eine Gruppe Ar oder Ar-$C_{1-3}$-Alkyl oder gegebenenfalls durch Y unterbrochenes und gegebenenfalls durch Z substituiertes $C_{1-7}$-Alkyl sind, oder |
| $R^4$ mit $R^5$ | zusammen mit dem N-Atom, an dem sie gebunden sind, einen 4- bis 6-gliedrigen gesättigten Ring bildet, |
| Y | Sauerstoff, Schwefel oder eine Gruppe $N(R^6)$, $C(O)N(R^6)$ oder $N(R^6)C(O)$, |
| Z | eine Gruppe -(O oder S)-$R^7$, -$N(R^7,R^8)$, -$C(O)N(R^7,R^8)$ oder -$N(R^7)C(O)R^8$, |
| n | die Zahl 1 oder 0 ist, wobei falls n die Zahl 1 ist, $R^5$ Wasserstoff ist, |
| Ar | durch 1 bis 3 Gruppen $R^9$ oder $OR^9$ substituiertes Phenyl, und |
| $R^6$ bis $R^9$ | Wasserstoff oder $C_{1-3}$-Alkyl sind, |

und von Salzen der Oxetanone der Formel I, worin X nicht Wasserstoff ist, mit schwachen Säuren.

Ferner betrifft die Erfindung neue im besagten Verfahren vorkommende Zwischenprodukte.

Verbindungen der Formel I sind bekannt, z.B. aus EP 185 359A2. Sie zeichnen sich durch wertvolle pharmakologische Eigenschaften aus. Sie hemmen insbesondere die Pankreaslipase und können demgemäss zur Bekämpfung oder Verhütung von Krankheiten, insbesondere von Obesitas, Hyperlipämie, Atherosklerose und Arteriosklerose, Verwendung finden.

Das erfindungsgemässe Verfahren ist dadurch gekennzeichnet, dass man

a) ein β-Hydroxy-δ-lacton der Formel

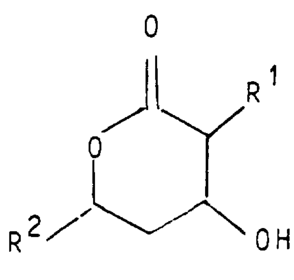

II

veräthert,

b) den erhaltenen Aether der Formel

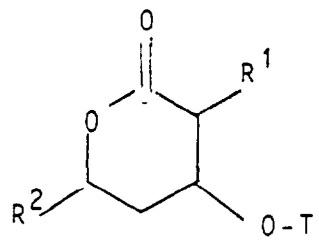

III

worin T eine leicht spaltbare Aethergruppe ist, mit einer Base öffnet,

c) das erhaltene Salz der Formel

$$R^2\text{-CHOHCH}_2\text{CH(O-T)CH(R}^1\text{)COO-M} \quad \text{IV}$$

worin M ein Alkali- oder Erdalkalimetall ist, in beliebiger Reihenfolge mit einem Arylmethylhalogenid und einer Base umsetzt und

d) den entstandenen Diäther der Formel

$$R^2\text{-CH(OCH}_2\text{-Ar)CH}_2\text{CH(O-T)CH(R}^1\text{)COO-M} \quad \text{V}$$

mit einer Säure selektiv spaltet,

e) die erhaltene β-Hydroxysäure der Formel

$$R^2\text{-CH(OCH}_2\text{-Ar)CH}_2\text{CHOHCH(R}^1\text{)COOH} \quad \text{VI}$$

gegebenenfalls nach Aufspaltung in ihre Enantiomeren cyclisiert,

f) den erhaltenen β-Lactonäther der Formel

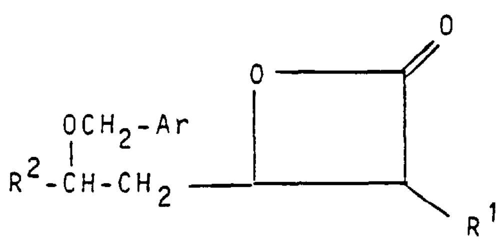

VII

spaltet, und

g) den erhaltenen β-Lactonalkohol der Formel I, worin X Wasserstoff ist, gegebenenfalls mit einem die Gruppe X einführenden Mittel verestert und

h) den erhaltenen Ester, gegebenenfalls in Form eines Salzes mit einer schwachen Säure, isoliert.

In der EPA 0 189 577, in Helv. Chim. Acta 70 (1987) 1412-8 und in J. Org. Chem. 53 (1988) 1218-21 werden nicht-aromatische Aether, wie die THP-Aether der Formeln V, 23 bzw. 15 zu den Oxetanonen der Formeln IV, 24 bzw. 16 cyclisiert. Im Gegensatz hierzu werden im Verfahren der vorliegenden Erfindung die Arylmethyläther, z.B. Benzyläther, der Formel VI zu den Oxetanonen VII cyclisiert. Zur Bestimmung der Konfiguration am C(3) und C(4) der in Helv. Chim.

Acta 70 (1987) 196-202 beschriebenen β-Lactone wird eines davon (der Formel 20) in das entsprechende δ-Lacton (der Formel 25) übergeführt. Hingegen werden im vorliegenden Verfahren δ-Lactone, wie diejenigen der Formel II, in β-Lactone der Formel VII übergeführt. Einige der in CA 110(1989) 134949q und in der DOS 24 00 429 genannen β-Keto-δ-lactone fallen unter die vorliegenden Formeln XII bzw. XXI. Hingegen ist keine der weiter unten als neu aufgezählten, ebenfalls unter diese Formeln fallenden Verbindungen in den genannten CA und DOS spezifisch beschrieben.

Alkylgruppen sind geradkettige oder verzweigte Kohlenwasserstoffreste, wie Methyl, Aethyl, Propyl, Isopropyl, Butyl, Isobutyl, Pentyl, Hexyl, Undecyl, Hexadecyl und Heptadecyl. Beispiele von Arylgruppen sind Phenyl, Tolyl und Xylyl. Beispiele von 4- bis 6-gliedrigen gesättigten Ringen sind Pyrrolidinyl und Pyridinyl.

Beispiele von schwachen Säuren, die mit den Verbindungen der Formel I Salze bilden können, sind p-Toluolsulfonsäure, Methansulfonsäure, Oxalsäure, Ascorbinsäure, Fumarsäure, Maleinsäure, Aepfelsäure, Citronensäure und Phosphorsäure.

Beispiele von leicht spaltbaren Aethergruppen sind Tetrahydro-2H-pyran-2-yl (THP), 1-Aethoxyäthyl oder Silyläthergruppen, wie Tri-$C_{1-4}$-alkyl- oder Mono-(aryl-$C_{1-4}$-alkyl)-di-($C_{1-4}$-alkyl)-silylgruppen, z.B. t-Butyldimethylsilyl.

Ein THP-Aether der Formel III lässt sich herstellen, indem man ein β-Hydroxy-δ-lacton der Formel II in einem Lösungsmittel, wie t-Butylmethyläther (TBME), Tetrahydrofuran (THF) oder Toluol, in Gegenwart katalytischer Mengen Säure, wie Pyridinium-p-toluolsulfonat oder p-Toluolsulfonsäure, mit 3,4-Dihydro-2H-pyran bei etwa 50°C umsetzt. Der THP-Aether III lässt sich anschliessend mittels Natron- oder Kalilauge öffnen.

Einen Silyläther III kann man herstellen durch Umsetzung des β-Hydroxy-δ-lactons II mit einem Silylhalogenid, wie dem t-Butyldimethylsilylchlorid, in Gegenwart einer Base, wie Aethyldiisopropylamin, in einem Lösungsmittel, wie Dimethylformamid (DMF) unter Erhitzen, z.B. auf 50-100°C. Die Oeffnung eines Silyläthers III lässt sich mit einer alkalischen oder erdalkalischen Base, wie Kalilauge, in einem Lösungsmittel, wie Dioxan, bewerkstelligen.

Als Base bei der Verätherung des Alkali- oder Erdalkalimetallsalzes der Formel IV zum Diäther V kann man Alkalimetallhydride, wie NaH, oder vorzugsweise Alkalimetall-t-butylate, zweckmässig das Na-t-Butylat, verwenden. Diese Verätherung lässt sich so bewerkstelligen, dass man das Salz IV z.B. mit Benzylbromid und NaH oder Na-t-Butylat in einem Lösungsmittel, wie THF oder TBME, behandelt.

Ein Diäther V lässt sich mit einer Säure, wie Salzsäure im Fall eines THP-Aethers oder Essigsäure im Fall eines Silyläthers, bei einer Temperatur bis zu 60°C selektiv spalten.

Die fakultative Aufspaltung einer racemischen β-Hydroxysäure VI kann mittels eines chiralen Amins, wie (R)-(+)- oder (S)-(-)-α-Methylbenzylamin in einem Lösungsmittel, wie einem Ester, z.B. Essigsäuremethyl- oder -äthylester, erfolgen.

Eine β-Hydroxysäure VI kann man mit einem Arylsulfonylhalogenid, wie Benzolsulfochlorid, in einem Lösungsmittel, wie Pyridin, unter Kühlung bis auf -10°C, cyclisieren.

Die Spaltung des β-Lactonäthers VII lässt sich durch Hydrierung in einem Lösungsmittel, wie einem Kohlenwasserstoff oder Halokohlenwasserstoff, z.B. Hexan oder Methylenchlorid, einem Ester oder Aether, z.B. Aethylacetat oder THF, über einem Katalysator wie Palladium auf Kohle (Pd/C) bei einer Temperatur bis zu etwa 40°C durchführen.

Die fakultative Veresterung eines β-Lactonalkohols der Formel I, worin X Wasserstoff ist, mit einer Säure der Formel $(R^3,R^4)NCH(R^5)(CH_2)_n$-COOH lässt sich in Gegenwart von Triphenylphosphin und von Azodicarbonsäurediäthylester in einem Lösungsmittel, wie einem Aether, z.B. THF, bei einer Temperatur bis zu etwa -15°C bewerkstelligen.

Eine β-Hydroxysäure der obigen Formel VI lässt sich auch dadurch herstellen, dass man

a) ein Salz der obigen Formel IV mit einem Halogenid der Formel $R^{10}$-Hal, worin $R^{10}$ $C_{1-4}$-Alkyl oder Aryl-$C_{1-4}$-alkyl ist, verestert,

b) den erhaltenen Ester der Formel

$$R^2\text{-CHOHCH}_2\text{CH(O-T)CH(}R^1\text{)COO-}R^{10} \qquad \text{IV-A}$$

veräthert und

c) den erhaltenen Diäther der Formel

$$R^2\text{-CH(OCH}_2\text{-Ar)CH}_2\text{CH(O-T)CH(}R^1\text{)COO-}R^{10} \qquad \text{V-A}$$

in beliebiger Reihenfolge verseift und in β-Stellung spaltet.

Die Veresterung eines Salzes IV mit einem Halogenid $R^{10}$-Hal, z.B. mit Benzylbromid, kann man in einem Lösungsmittel, wie THF, durchführen.

Die Verätherung des Alkoholesters IV-A kann mit einem Chlorid der Formel Ar-$CH_2OC(NH)CCl_3$, z.B. mit Benzyl-2,2,2-trichloracetimidat, in Gegenwart einer Säure, wie Trifluormethansulfonsäure, in einem Lösungsmittel, wie Cyclohexan, Hexan oder Methylenchlorid, erfolgen.

Einen Diäther V-A kann man zunächst mit einer Säure, wie wässrige Essigsäure im Fall eines Silyläthers oder wie Salzsäure im Fall eines THP-Aethers, in einem Lösungsmittel, wie Dioxan, bei einer Temperatur bis zur Rückflusstemperatur des Reaktionsgemisches behandeln und anschliessend in einem Lösungsmittel, wie einem Alkanol, z.B. Methanol, mittels einer starken Base, wie einem Alkali- oder Erdalkalimetallhydroxid, z.B. Kaliumhydroxid, bei einer Temperatur bis zu etwa 70°C verseifen.

Zur Herstellung einer β-Hydroxysäure VI kann man auch so vorgehen, dass man

a) ein β-Hydroxy-δ-lacton II verestert,

b) den erhaltenen Ester der Formel

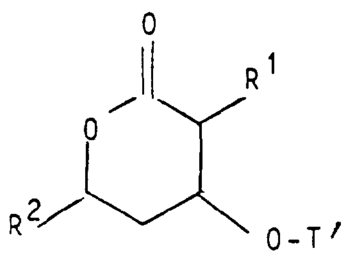

III'

worin T' Aroyl ist,
säurekatalytisch in Gegenwart eines Alkohols der Formel $R^{10}$-OH zu einem Ester der Formel

$$R^2\text{-CHOHCH}_2\text{CH(O-T')CH(R}^1\text{)COO-R}^{10} \qquad \text{IV-B}$$

öffnet,

c) den Ester der Formel IV-B veräthert und

d) den erhaltenen Aetherdiester der Formel

$$R^2\text{-CH(OCH}_2\text{-Ar)CH}_2\text{CH(O-T')CH(R}^1\text{)COO-R}^{10} \qquad \text{V-B}$$

doppelt verseift.

Die Veresterung des β-Hydroxy-δ-lactons der Formel II kann man z.B. mit einem funktionellen Derivat einer Säure der Formel Ar-COOH, z.B. einem Säurehalogenid oder Säureanhydrid, wie Benzoesäureanhydrid, und einer starken Säure, wie Perchlorsäure, oder einer Base, wie Dimethylaminopyridin, als Katalysator in einem Lösungsmittel, wie Toluol, und die anschliessende Oeffnung des Esters III' säurekatalytisch z.B. in Gegenwart einer Säure, wie Schwefelsäure oder Salzsäure, mit einem Alkohol der Formel $R^{10}$-OH, z.B. einem niederen Alkanol, wie Methanol, gegebenenfalls in einem Lösungsmittel, wie Toluol, bei einer Temperatur bis zu 60°C durchführen.

Die Verätherung des Alkoholesters IV-B kann in Analogie zur weiter oben beschriebenen Verätherung des Alkoholesters IV-A bewerkstelligt werden.

Die Verseifung eines Aetherdiesters V-B lässt sich in einem Lösungsmittel, wie einem Alkanol, z.B. Methanol, mittels einer starken Base, wie einem Alkali- oder Erdalkalimetallhydroxid, z.B. Kaliumhydroxid, bei einer Temperatur bis zu etwa 70°C durchführen.

Die β-Hydroxy-δ-lactone der Formel II lassen sich dadurch herstellen, dass man

a) einen β-Hydroxyester der Formel

$$R^2\text{-CHOHCH}_2\text{COO-R} \qquad \text{VIII}$$

worin R $C_{1-4}$ -Alkyl ist,
verseift,

b) das Imidazolid der erhaltenen β-Hydroxysäure der Formel

$$R^2\text{-CHOHCH}_2\text{COOH} \qquad \text{IX}$$

mit dem Mg-Salz des Malonsäureesterderivats der Formel

$$\text{HOCOCH}(R^1)\text{COO-R} \qquad \text{X}$$

umsetzt,

c) den entstandenen δ-Hydroxy-β-ketoester der Formel

$$R^2\text{-CHOHCH}_2\text{COCH}(R^1)\text{COO-R} \qquad \text{XI}$$

cyclisiert und

d) das erhaltene β-Keto-δ-lacton der Formel

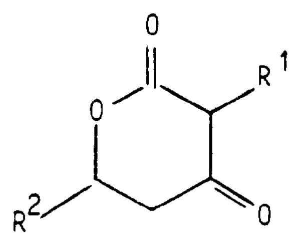

XII

katalytisch hydriert.

Die Verseifung des β-Hydroxyesters der Formel VIII kann man in einem Lösungsmittel, wie Dioxan, mit einer Base, wie Natriumhydroxid in einem Lösungsmittel, wie Methanol, durchführen.

Das Magnesiumsalz eines Malonsäureesterderivats der Formel X kann man herstellen durch Umsetzung des Malonsäurediesters der Formel $CH_2(COOR)_2$ mit einer Lösung von Natriummethylat in Methanol und mit einem Halogenid der Formel $R^1$-Hal, z.B. dem Bromid, bei einer Temperatur bis zur Rückflusstemperatur des Lösungsmittels. Das erhaltene Malonsäurediesterderivat der Formel $R^1$-$CH(COOR)_2$ wird dann mit einem Alkalimetallhydroxid, z.B. Kaliumhydroxid, in einem nieder-Alkanol R-OH, wie Methanol, zum Monoester der Formel X hydrolysiert und letzterer mit Magnesiumchlorid in THF in Gegenwart von Triäthylamin bei 0°C zum erwünschten Magnesiumsalz umgesetzt.

Das Imidazolid der β-Hydroxysäure der Formel IX lässt sich herstellen, indem man diese in THF mit 1,1'-Carbonyldiimidazol umsetzt.

Das Magnesiumsalz des Monoesters der Formel X kann man bei Raumtemperatur mit dem Imidazolid der β-Hydroxysäure der Formel IX zum δ-Hydroxy-β-ketoester der Formel XI umsetzen.

Letzteren kann man in einem Lösungsmittel, wie Aethylacetat, mit einer Säure, wie Salzsäure, oder einer Base, wie Natronlauge, zum β-Keto-δ-lacton der Formel XII cyclisieren.

Die katalytische Hydrierung dieses Lactons zum β-Hydroxy-δ-lacton der Formel II kann man in einem Lösungsmittel, wie Aethylacetat, oder einem Aether, wie THF, in Gegenwart von Raney-Nickel durchführen.

Die β-Hydroxy-δ-lactone der Formel II lassen sich auch dadurch herstellen, dass man

a) einen β-Ketoester der Formel

$$CH_3COCH(R^1)COO\text{-}R \qquad XIII$$

mit einem Ester der Formel

$$R^2\text{-}COO\text{-}R \qquad XIV$$

umsetzt,

b) den erhaltenen Diketoester der Formel

$$R^2\text{-}COCH_2COCH(R^1)COO\text{-}R \qquad XV$$

cyclisiert und

c) das erhaltene Pyron der Formel

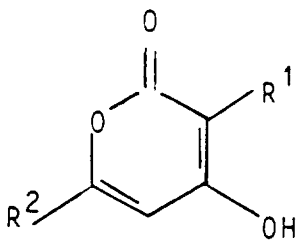

XVI

katalytisch hydriert.

Einen β-Ketoester der Formel XIII kann man herstellen durch Alkylierung des entsprechenden β-Ketoesters der Formel $CH_3COCH_2COOR$ mit einem Halogenid der Formel $R^1$-Hal, z.B. dem Bromid, in einer methanolischen Natriummethylatlösung bei einer Temperatur bis zur Rückflusstemperatur des Reaktionsgemisches.

Die Reaktion des β-Ketoesters der Formel XIII mit einem Ester der Formel XIV kann man in Gegenwart von Natriumhydrid in einem Lösungsmittel, wie THF, und in Gegenwart von Butyllithium in Hexan unter Abkühlen, z.B. bei -10°C durchführen.

Die Cyclisierung zu einem Pyron der Formel XVI kann man in einem Lösungsmittel, wie Toluol, in Gegenwart von 1,8-Diazabicyclo[5.4.0]undec-7-en(1,5,5) (DBU) bewerkstelligen.

Das Pyron der Formel XVI kann man in der gleichen Weise wie oben für die katalytische Hydrierung des β-Keto-δ-lactons der Formel XII beschrieben zum β-Hydroxy-δ-lacton der Formel II hydrieren.

Ein β-Keto-δ-lacton der Formel XII kann man auch dadurch herstellen, dass man

a) einen β-Ketoester der obigen Formel

$$CH_3COCH(R^1)COO\text{-}R \qquad XIII$$

mit einem Aldehyd der Formel

$$R^2\text{-}CHO \qquad XVII$$

umsetzt und

b) den erhaltenen δ-Hydroxy-β-ketoester der Formel XI cyclisiert.

Die Reaktion des β-Ketoesters der Formel XIII mit dem Aldehyd der Formel XVII kann man in der gleichen Weise durchführen wie die weiter oben beschriebene Umsetzung mit dem Ester der Formel XIV.

Die Cyclisierung des δ-Hydroxy-β-ketoesters der Formel XI zum β-Keto-δ-lacton der Formel XII lässt sich mit Wasser zweckmässig bei Raumtemperatur bewerkstelligen.

Ein β-Keto-δ-lacton der Formel XII kann man ferner dadurch herstellen, dass man

a) einen β-Hydroxyester der obigen Formel

$$R^2\text{-CHOHCH}_2\text{COO-R} \qquad \text{VIII}$$

veräthert,

b) den erhaltenen Aether der Formel

$$R^2\text{-CH(OCH}_2\text{-Ar)CH}_2\text{COO-R} \qquad \text{XVIII}$$

verseift,

c) die erhaltene Aethersäure der Formel

$$R^2\text{-CH(OCH}_2\text{-Ar)CH}_2\text{COOH} \qquad \text{XIX}$$

halogeniert,

d) das erhaltene Säurehalogenid mit Meldrumsäure umsetzt,

e) die erhaltene Verbindung der Formel

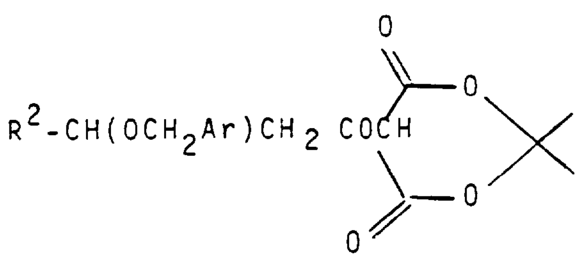

XX

hydrogenolysiert und zu einem β-Keto-δ-lacton der Formel

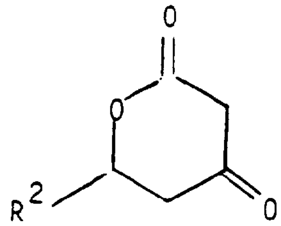

XXI

cyclisiert, und

f) das β-Keto-δ-lacton der obigen Formel XXI mit einem die Gruppe $R^1$ bzw. $-CH_2-R^{11}$ einführenden Aldehyd der

Formel

$$R^{11}\text{-CHO} \qquad XXII$$

worin $R^{11}$ zusammen mit der Methylengruppe die Gruppe $R^1$ darstellt,

zu einem β-Keto-δ-lacton der obigen Formel XII umsetzt.

Die Verätherung des β-Hydroxyesters der Formel VIII zum Aether der Formel XVIII kann man in einem Lösungsmittel, wie Cyclohexan, z.B. mit Benzyltrichloracetimidat in Gegenwart von Trifluormethansulfonsäure bei einer Temperatur bis zu 30°C durchführen.

Die Verseifung des Aethers der Formel XVIII zur Aethersäure der Formel XIX lässt sich mit einem Alkalimetallhydroxid, wie Kaliumhydroxid, in einem Lösungsmittel, wie Methanol, bewerkstelligen.

Die Halogenierung der Aethersäure der Formel XIX kann man z.B. mit Oxalylchlorid in einem Lösungsmittel, wie Methylenchlorid, bei einer Temperatur bis zu etwa 25°C durchführen.

Die Umsetzung des erhaltenen Säurehalogenids mit Meldrumsäure kann man in einem Lösungsmittel, wie Methylenchlorid, in Gegenwart von einer Base, wie Pyridin, unter Abkühlen bis auf -10°C durchführen.

Die Hydrogenolyse und Cyclisierung der Verbindung der Formel XX zum β-Keto-δ-lacton der Formel XXI kann in einem Lösungsmittel, wie Aethylacetat, mittels eines Katalysators, wie Pd/C erfolgen.

Die Reaktion des β-Keto-δ-lactons der Formel XXI mit dem Aldehyd der Formel $R^{11}$-CHO zum β-Keto-δ-lacton der Formel XII erfolgt z.B. mit einem Boranamin-Komplex, wie Boran-triäthylamin, Boran-trimethylamin oder Boran-morpholin, in einem Lösungsmittel, wie Methanol, bei einer Temperatur bis zu 50°C.

Ein β-Keto-δ-lacton der Formel XXI kann man auch dadurch herstellen, dass man

a) das Imidazolid der β-Hydroxysäure der obigen Formel

$$R^2\text{-CHOHCH}_2\text{COOH} \qquad IX$$

mit dem Mg-Salz eines Malonsäuremononiederalkylesters umsetzt und

b) den entstandenen δ-Hydroxy-β-ketoester der Formel

$$R^2\text{-CHOHCH}_2\text{COCH}_2\text{COO-R} \qquad XXIII$$

zum β-Keto-δ-lacton der obigen Formel XXI cyclisiert.

Die Herstellung des Mg-Salzes eines Malonsäuremononiederalkylesters, die Umsetzung dieses Salzes mit dem Imidazolid der β-Hydroxysäure der Formel IX zum δ-Hydroxy-β-ketoester der Formel XXIII und dessen Cyclisierung zum β-Keto-δ-lacton der Formel XXI kann man durchführen wie weiter oben beschrieben für die Herstellung des β-Hydroxy-δ-lactons der Formel II über das Imidazolid der β-Hydroxysäure der Formel IX und das Mg-Salz des Malonsäureesterderivats der Formel X.

Eine bevorzugte Ausführungsform des Verfahrens der vorliegenden Erfindung ist dadurch gekennzeichnet, dass man einen β-Ketoester XIII mit einem Aldehyd XVII umsetzt, den erhaltenen δ-Hydroxy-β-ketoester XI zum β-Keto-δ-lacton XII cyclisiert, letzteres katalytisch hydriert und das erhaltene β-Hydroxy-δ-lacton II nach dem weiter oben beschriebenen Verfahren über die Verbindungen der Formeln III bis VII in einen Ester I überführt.

Besonders bevorzugt ist dabei die Herstellung des Esters der Formel I, worin $R^1$ n-Hexyl und $R^2$ Undecyl ist.

Die folgenden unter die Formeln II, XII bzw. XVI fallenden β-Hydroxy-δ-lactone, β-Keto-δ-lactone und Pyrone sind neu und als solche Gegenstand der vorliegenden Erfindung:

rac-(2RS,3RS,5SR)-2-Hexyl-3-hydroxy-5-undecyl-δ-valeriolacton,
rac-(2RS,3RS,5SR)-2-Aethyl-5-heptadecyl-3-hydroxy-δ-valeriolacton,
(2S,3S,5R)-2-Aethyl-5-heptadecyl-3-hydroxy-δ-valeriolacton und
rac-(2RS,3RS,5SR)-2-Hexyl-3-hydroxy-5-pentyl-δ-valeriolacton;
rac-5,6-Dihydro-3-hexyl-4-hydroxy-6-undecyl-2H-pyran-2-on,
(R)-3-Aethyl-5,6-dihydro-6-heptadecyl-4-hydroxy-2H-pyran-2-on und

rac-5,6-Dihydro-3-hexyl-4-hydroxy-6-pentyl-2H-pyran-2-on;
3-Hexyl-4-hydroxy-6-undecyl-2H-pyran-2-on,
3-Aethyl-6-heptadecyl-4-hydroxy-2H-pyran-2-on und
3-Hexyl-4-hydroxy-6-pentyl-2H-pyran-2-on.

Die β-Keto-δ-lactone der Formel XXI, worin $R^2$ die weiter oben angegebene Bedeutung hat, die Anzahl C-Atome in der Alkylgruppe $R^2$ jedoch mehr als 9 betragen soll, insbesondere die folgenden:

(R)-5,6-Dihydro-6-undecyl-2H-pyran-2,4(3H)-dion und
(R)-5,6-Dihydro-6-heptadecyl-2H-pyran-2,4(3H)-dion

sind ebenfalls neu und Gegenstand der Erfindung.

Beispiel 1

a) Unter Stickstoff und Rühren werden zu 720 g 30%iger Natriummethylatlösung in 1200 ml Methanol 465 g Acetessigsäuremethylester zugetropft. Dann werden 727 g 1-Bromhexan zugegeben und das Reaktionsgemisch wird während 20 Stunden am Rückfluss gekocht. Die Hauptmenge des Methanols wird abdestilliert und der Rückstand auf Eiswasser gegossen. Das Gemisch wird mit n-Hexan und dann mit Wasser extrahiert. Die organischen Phasen werden vereinigt und über Natriumsulfat getrocknet. Das Lösungsmittel wird abgedampft und der Rohester destilliert. Man erhält 499,4 g 2-Acetyloctansäuremethylester, Sdp. 124-128°/15 Torr.

b) 200,3 g 2-Acetyloctansäuremethylester werden zu einer Suspension von 26,4 g Natriumhydrid in 1250 ml THF gegeben. Nach 1 Stunde Rühren bei 0-5° wird auf -10° abgekühlt. Bei dieser Temperatur werden 675 ml 1,56M Butyllithium in Hexan zugegeben. Nach 30 Minuten Rühren bei -10° werden 107,2 g Laurinsäuremethylester zugetropft. Es wird 1 Stunde bei -10° weitergerührt. Die Reaktionslösung wird unter Argon zu 250 ml 37% Salzsäure und 300 g Eis gegeben. Es wird mit Hexan und Wasser extrahiert. Die vereinigten organischen Phasen werden getrocknet, filtriert und eingedampft.

Der Rückstand (290,5 g) wird in 1250 ml Toluol gelöst, mit 76,1 g DBU versetzt und unter Argon 30 Minuten am Rückfluss gekocht. Die Reaktionslösung wird in Toluol mit 3N Salzsäure und Wasser extrahiert. Die vereinigten Toluol-Phasen werden bei 40° eingedampft. Das Produkt wird in Hexan gelöst und unter Rühren auf Raumtemperatur abgekühlt. Nach 17 Stunden Rühren bei -10° wird das Kristallisat abgenutscht, mit Hexan gewaschen und getrocknet. Es resultierten 123,9 g (70,0%) 3-Hexyl-4-hydroxy-6-undecyl-2H-pyran-2-on, Smp. 83-84°.

c) Zu 100 g des Pyrons von b) werden 100 g Raney-Nickel und 2000 ml Essigsäureäthylester gegeben. Nach 17 Stunden Hydrierung unter Rühren bei 30° wird der Katalysator abgenutscht und mit Essigsäureäthylester gewaschen. Das Filtrat wird eingeengt und über Nacht bei -10° gerührt. Das Kristallisat wird abgenutscht, mit Essigsäureäthylester gewaschen und dann getrocknet. Es resultierten 90,7 g (89,7%) rac-(2RS,3RS,5SR)-2-Hexyl-3-hydroxy-5-undecyl -δ-valeriolacton, Smp. 98-99°.

d) Unter Rühren werden 177,3 g des δ-Lactons von c) in 1250 ml Toluol suspendiert. Nach Zugabe von 138,6 g Benzoesäureanhydrid wird 10 Minuten gerührt. Dann werden 2,5 ml Perchlorsäure zugegeben. Es wird 2,5 Stunden weitergerührt. Die Reaktionslösung wird in Toluol mit 1N Natronlauge und dann mit Wasser extrahiert. Die vereinigten Toluol-Phasen werden getrocknet, das Trocknungsmittel abgenutscht und mit Toluol gewaschen. Abdampfen des Lösungsmittels ergibt 237,2 g (103,4%) rac-(2RS,3RS,5SR)-3-Benzoyloxy-2-hexyl -5-undecyl-δ-valeriolacton, welches ohne Reinigung in der nächsten Stufe eingesetzt wurde.

e) Unter Argon werden 236 g des Benzoats von d) in 1250 ml Methanol unter Rühren mit 2,5 ml konz. Schwefelsäure versetzt und während 18 Stunden weitergerührt. Anschliessend wird das pH der Reaktionslösung mit Triäthylamin auf 9 eingestellt und das Methanol abgedampft. Der Rückstand wird in Hexan aufgenommen, mit Wasser gewaschen und die Wasserphase mit Hexan extrahiert. Nach dem Trocknen der vereinigten Hexanphasen wird filtriert. Das Filtrat wird vom Hexan befreit. Es resultierten 259 g (105,5%) rac-(2RS,3RS,5SR)-3-Benzoyloxy-2-hexyl -5-hydroxyhexadecansäuremethylester, welches ohne Reinigung in der nächsten Stufe eingesetzt wird.

f) Unter Argon werden 258 g des Hydroxyesters von e) in 1250 ml Hexan unter Rühren mit 152 g Benzyl-2,2,2-trichloracetimidat versetzt. Dann werden 3,2 ml Trifluormethansulfonsäure zugegeben. Nach 17 Stunden Rühren wird der Niederschlag abgenutscht und mit Hexan gewaschen. Das Filtrat wird mit 5%iger Natriumbicarbonatlösung und dann mit Wasser extrahiert. Die Hexanphase wird getrocknet, filtriert und eingedampft. Es resultierten

320 g (110%) rac-(2RS,3RS,5SR)-3-Benzoyloxy-5-benzyloxy -2-hexylhexadecansäuremethylester.

g) Unter Argon werden 319 g des Benzyläthers von f) in 1125 ml Methanol mit eine Lösung von 140 g Kaliumhydroxid in 125 ml Wasser versetzt. Das Reaktionsgemisch wird bei 40° während 17 Stunden gerührt und anschliessend eingeengt. Die Suspension wird zusammen mit n-Hexan mit 10%iger Natriumchloridlösung und dann 1N Salzsäure extrahiert. Die vereinigten organischen Phasen werden mit Natriumsulfat getrocknet, das Trocknungsmittel wird abgenutscht und mit Hexan gewaschen. Das Filtrat wird auf 1000 ml eingeengt und 1 Tag bei -20° gehalten. Das Kristallisat wird abgenutscht, mit n-Hexan gewaschen und verworfen. Das Filtrat ergibt nach Abdampfen 169 g (73%) rac-(2RS,3RS,5SR)-5-Benzyloxy-2-hexyl -3-hydroxyhexadecansäure.

h) Unter Argon werden unter Rühren zu 169 g der β-Hydroxysäure von g) in 1250 ml Essigsäuremethylester und 39,4 g (S)-(-)-α-Methylbenzylamin zugetropft. Die Lösung wird mit Phenäthylaminsalz angeimpft und dann auf -10° abgekühlt. Nach 17 Stunden bei -10° wird der Kristallbrei abgenutscht, mit Essigsäuremethylester gewaschen, abgesaugt und getrocknet. Nach zwei weiteren Kristallisationen aus Essigsäuremethylester erhält man 56,5 g (19,4%) des Phenäthylamisalzes der (2S,3S,5R)-5-Benzyloxy-2-hexyl-3-hydroxyhexadecansäure, Smp. 104-105°.

i) Es werden 56,5 g des Phenäthylaminsalzes von h) unter Rühren mit 565 ml Hexan und 120 ml 1N Salzsäure versetzt. Die organische Phase wird mit Wasser gewaschen, getrocknet und eingeengt. Man erhält 44,2 g (98,8%; 19,1% bezogen auf das δ-Lacton von c)) (2S,3S,5R)-5-Benzyloxy-2-hexyl-3-hydroxyhexadecansäure.

j) Unter Rühren werden 231,5 g der β-Hydroxysäure von i) in 2500 ml Pyridin gelöst und auf 0° gekühlt. Nun werden 176,6 g Benzolsulfochlorid zugetropft. Die Lösung wird während 20 Stunden bei 0° weitergerührt. Danach wird Wasser zugegeben und 30 Minuten bei Raumtemperatur gerührt. Das Pyridin wird abgedampft. Der Kristallbrei wird zusammen mit Hexan nacheinander mit 2N Salzsäure, 5%iger Natriumbicarbonatlösung und 10%iger Natriumchloridlösung extrahiert. Die Hexanphasen werden vereinigt und eingeengt. Nach dem Trocknen wird Aktivkohle zugegeben, 1 Stunde gerührt, abgenutscht, mit Hexan gewaschen und eingedampft. Es resultierten 222,1 g (99,9%) (3S,4S)-4-[(R)-2-Benzyloxytridecyl]-3-hexyl-2-oxetanon, welches ohne Reinigung in der nächsten Stufe eingesetzt wird.

k) Es werden 222 g des β-Lactons von j) in 2500 ml THF gelöst und an 11 g Pd/C 10% während 18 Stunden hydriert. Die Lösung wird filtriert und mit THF gewaschen. Das Filtrat wird abgedampft. Die erhaltenen Kristalle werden in Hexan gelöst. Nach 18 Stunden Rühren bei 5° wird das Kristallisat abgenutscht, mit Hexan gewaschen und getrocknet. Man erhält 150,5 g (84,9%) (3S,4S)-3-Hexyl-4-[(R)-2-hydroxytridecyl]-2-oxetanon, Smp. 61-62°C.

Beispiel 2

Unter Rühren werden 88,6 g des Hydroxy-β-lactons von Beispiel 1k), 51,7 g N-Formyl-(S)-leucin und 98,4 g Triphenylphosphin in 1000 ml THF gelöst. Der auf -10°C kühlten Lösung wird eine Lösung von 72,6 g Azodicarbonsäurediäthylester in 250 ml THF zugetropft. Die Reaktionslösung wird 15 Stunden bei -10°C gerührt. Anschliessend wird das Lösungsmittel abgedampft. Der Kristallbrei wird unter Rühren zwischen Hexan und 70% Methanol/Wasser mehrmals verteilt. Die vereinigten Hexan-Phasen werden über Natriumsulfat getrocknet, das Trocknungsmittel wird abgenutscht und mit Hexan gewaschen. Nach Abdestillieren des Hexans wird das Rohprodukt in Hexan gelöst und langsam auf 5° abgekühlt. Das Kristallisat wird abgenutscht, mit Hexan gewaschen und getrocknet. Man erhält 98,0 g N-Formyl-L-leucin(S)-1-[[(2S,3S)-3-hexyl-4-oxo-2-oxetanyl]methyl]-dodecylester, Smp. 44-45°C.

Beispiel 3

a) Unter Stickstoff und Rühren wird eine Suspension von 206,7 g (R)-3-Hydroxytetradecansäuremethylester in 1000 ml Cyclohexan mit 214,2 g Benzyltrichloracetimidat versetzt und bei 20° gelöst. Unter Kühlen im Eis/Wasserbad werden 10 ml Trifluormethansulfonsäure so zugetropft, dass die Temperatur zwischen 20-23° bleibt. Die entstandene Suspension wird bei 25-30° gerührt. Der Niederschlag wird filtriert, der Filterkuchen mit Cyclohexan gewaschen, die Filtrate mit ges. $NaHCO_3$-Lösung, Wasser und ges. NaCl-Lösung extrahiert. Die organische Phase wird über Magnesiumsulfat getrocknet, filtriert, der Filterkuchen mit Cyclohexan gewaschen und die Filtrate eingedampft. Man erhält 314,5 g (R)-3-(Benzyloxy)-tetradecansäuremethylester.

b) Einer Lösung von 89,8 g Kaliumhydroxid in 680 ml Methanol werden 314,0 g des Methylesters von a) zugegeben. Nach 18,5 Stunden Rühren bei Raumtemperatur wird die entstandene Suspension auf 680 g Eis gegossen und

unter Kühlen bei 0-7° mit 200 ml 25-proz. wässriger HCl-Lösung auf pH 1 gestellt. Die entstandene Emulsion wird mit Methylenchlorid extrahiert. Die organische Phase wird über Magnesiumsulfat getrocknet, filtriert, der Filterkuchen mit Methylenchlorid gewaschen und die Filtrate eingedampft. Man erhält 277,3 g (R)-3-Benzyloxytetradecansäure.

c) Einer Lösung von 276,5 g der Säure von b) in 1400 ml Methylenchlorid werden 82,5 ml Oxalylchlorid zugetropft. Nach 3,5 Stunden Rühren wird die erhaltene Lösung von (R)-3-Benzyloxytetradecansäurechlorid auf 450 ml eingeengt.

d) Einer Lösung von 122,8 g Meldrumsäure in 900 ml Methylenchlorid werden bei -6 bis 0° 164 ml Pyridin zugetropft. Nach 10 Minuten Rühren bei -3 bis -1° wird die Lösung des Säurechlorids von c) zugetropft. Die entstandene Suspension wird 3 Stunden bei 0° gerührt, dann auf ein Gemisch von 400 g Eis und 1300 ml Salzsäure gegossen, 10 Minuten gerührt, die organische Phase abgetrennt und mit 300 ml 3N Salzsäure extrahiert. Die sauren wässrigen Phasen werden mit 500 ml Methylenchlorid extrahiert, die vereinigten organischen Phasen über Magnesiumsulfat getrocknet, filtriert, der Filterkuchen mit Methylenchlorid gewaschen und die Filtrate eingeengt. Die Lösung wird mit Kieselgel versetzt und gerührt. Das Kieselgel wird abfiltriert und mit Methylenchlorid gewaschen. Die Filtrate werden eingeengt. Man erhält 382,2 g 5-[(R)-3-Benzyloxy-1-hydroxytetradecyliden] -2,2-dimethyl-m-dioxan-4,6-dion.

e) Zu einer Lösung von 381,5 g des Produkts von d) in 2500 ml Essigester werden 33,1 g 5% Pd/C gegeben. Es wird 3,5 Stunden hydriert. Die Suspension wird filtriert, der Filterkuchen mit Essigester gewaschen und die Filtrate eingeengt. Die erhaltene Lösung wird bei 79-80° gekocht, auf Raumtemperatur gekühlt, eingedampft und getrocknet. Das Produkt wird in n-Hexan suspendiert, filtriert und der Filterkuchen mit n-Hexan gewaschen und die Kristalle werden getrocknet. Die Mutterlauge wird eingeengt, in n-Hexan gelöst und 72 Stunden bei 4° stehen gelassen. Die angefallenen Kristalle werden filtriert, mit n-Hexan gewaschen und getrocknet. Die beiden Kristallisate werden vereinigt, in Wasser suspendiert, gerührt, filtriert, der Filterkuchen mit Wasser gewaschen und die Kristalle werden getrocknet. Man erhält 62,1 g (R)-5,6-Dihydro-6-undecyl-2H-pyran-2,4(3H)-dion, Smp. 82-85°.

f) Unter Stickstoff werden einer Lösung von 43,14 g Boran-Triäthylamin-Komplex in 1000 ml Methanol 100,65 g des Pyrandions von e) zugesetzt. Es wird auf 40° erwärmt. Der Lösung werden 75,12 g Capronaldehyd zugetropft. Die Lösung wird 70 Minuten bei 41° gerührt, dann auf Raumtemperatur gekühlt und auf Eis/Wasser gegossen. Die Suspension wird unter Rühren mit 120 ml 3N Salzsäure versetzt und 30 Minuten gerührt. Die Kristalle werden genutscht, mit Wasser gewaschen und dann getrocknet. Das Produkt wird in n-Hexan suspendiert, 30 Minuten gerührt, genutscht, mit n-Hexan gewaschen und getrocknet. Man erhält 112,5 g (85%) (R)-5,6-Dihydro-3-hexyl-4-hydroxy-6-undecyl-2H-pyran-2-on, Smp. 106-108°, $[\alpha]_D^{20}$ = -45,6° (c = 1% in Dioxan).

g) Es werden 30,0 g des Pyranons von f) in 750 ml Aethylacetat gelöst und nach Zugabe von 30 g Raney-Nickel bei Raumtemperatur während 24,5 Stunden hydriert. Der Katalysator wird filtriert, mit Aethylacetat gewaschen und die Filtrate werden eingeengt. Das Produkt wird in Aethylacetat bei 45° gelöst, innert 2 Stunden auf 20°, dann innert 3 Stunden auf -10° gekühlt und bei dieser Temperatur 16 Stunden gerührt und dann filtriert. Die Kristalle werden mit Aethylacetat gewaschen und dann getrocknet. Man erhält 25,5 g (85%) (2S,3S,5R)-2-Hexyl-3-hydroxy-5-undecyl-δ-valeriolacton, Smp. 103-104,5°, $[\alpha]_D^{20}$ = +47,4° (c = 1% in $CHCl_3$).

h) Eine Lösung von 12,0 g des Lactons von g) in 70 ml DMF wird mit 6,77 g Aethyldiisopropylamin versetzt und nach Zugabe von 7,64 g t-Butyldimethylsilylchlorid während 13 Stunden unter Rühren auf 80° erhitzt. Das Reaktionsgemisch wird eingeengt, in 120 ml Hexan aufgenommen, filtriert und der Filterkuchen mit 50 ml Hexan gewaschen. Die vereinigten Filtrate werden mit 3N Salzsäure extrahiert und die organische Phase getrocknet. Nach Filtrieren und Einengen erhält man 15,7 g (2S,3S,5R)-3-t-Butyldimethylsiloxy-2-hexyl -5-undecyl-δ-valeriolacton.

i) Eine Lösung von 42,2 g des Lactons von h) in 570 ml Dioxan wird nach Zugabe von 90 ml 1N Kalilauge während 5,5 Stunden gerührt. Das Reaktionsgemisch wird eingeengt und durch Zusatz von Toluol und Destillation azeotrop getrocknet. Der Rückstand wird in 250 ml THF gelöst und nach Zugabe von 23,1 g Benzylbromid und 4,75 g 18-Crown-6 (1,4,7,10,13,16-Hexaoxacyclooctadecan) während 4 Stunden gerührt. Das Reaktionsgemisch wird eingeengt, mit n-Hexan versetzt und mit 3N Salzsäure extrahiert. Die organische Phase wird getrocknet, filtriert und eingeengt, wobei 61,7 g (2S,3S,5R)-3-t-Butyldimethylsiloxy-2-hexyl-5-hydroxyhexadecansäure-benzylester anfallen.

j) Eine Lösung von 60,6 g des Produkts von i) in 130 ml Methylenchlorid und 260 ml Cyclohexan wird mit 33,5 g

Benzyltrichloracetimidat versetzt und nach Zugabe von 0,53 ml Trifluormethansulfonsäure während 5 Stunden gerührt. Die Suspension wird filtriert, der Filterkuchen mit Hexan gewaschen und die vereinigten Filtrate der Reihe nach mit 3N Salzsäure, 3N Natronlauge, 3N Salzsäure und Wasser gewaschen. Die organische Phase wird getrocknet, filtriert und eingeengt, wobei 74,8 g (2S,3S,5R)-5-Benzyloxy-3-t-butyldimethylsiloxy -2-hexylhexadecansäure-benzylester anfallen.

k) Ein Gemisch von 20 g des Esters von j), 48 ml Eisessig, 16 ml Wasser und 16 ml Dioxan werden während 3,5 Stunden am Rückfluss erhitzt und anschliessend durch azeotrope Destillation unter Zusatz von 120 ml Dioxan von der Essigsäure befreit und eingeengt. Der Rückstand wird in Hexan gelöst und 14 Stunden bei -25° gelagert. Die gebildeten Kristalle werden filtriert, das Filtrat eingeengt und der in Methanol gelöste Rückstand mit einer wässrigen Lösung von Kaliumhydroxid während 24 Stunden durchmischt. Die Reaktionslösung wird eingedampft, der Rückstand in Hexan aufgenommen und mit 1N Salzsäure und 10% Kochsalzlösung gewaschen und eingeengt. Der Rückstand wird in Essigsäuremethylester gelöst und mit 2,6 g Benzylamin versetzt. Durch Kristallisation lassen sich 6,0 g (2S,3S,5R)-5-Benzyloxy-3-hydroxy-2-hexylhexadecansäure (des Produkts des Beispiels 1h) als Benzylaminsalz vom Smp. 57-60° und weitere 1,4 g vom Smp. 61-64° gewinnen.

Beispiel 4

a) Unter Stickstoff und Rühren werden zu einer Lösung von 129,2 g (R)-3-Hydroxytetradecansäuremethylester, 1033 ml Dioxan und 122,9 g 28-proz. Natronlauge zugetropft. Der Lösung werden 77,5 ml Methanol zugetropft. Nach weiteren 1,5 Stunden Rühren wird die entstandene Suspension filtriert und der Filterkuchen mit 1000 ml Dioxan gewaschen und abgesogen. Der Filterkuchen wird durch Zugabe von 650 ml 1,5N Salzsäure auf pH 0 gestellt. Die Suspension wird gerührt, filtriert, der Filterkuchen mit 3000 ml Wasser gewaschen und die Kristalle getrocknet. Man erhält 119,64 g (98%) (R)-3-Hydroxytetradecansäure, Smp. 70,6-71,4°.

b) Es werden unter Stickstoff und Rühren 5,11 g Magnesiumchlorid in 50 ml THF suspendiert und auf 0° gekühlt. Eine Lösung von 11,33 g Malonsäuremonomethylester in 70 ml THF und anschliessend 10,7 g Triäthylamin werden zugetropft. Die Suspension wird bei 0° gerührt.

c) Unter Stickstoff und Rühren werden zu einer Lösung von 7,33 g (R)-3-Hydroxytetradecansäure in 60 ml THF 6,81 g 1,1'-Carbonyldiimidazol zugegeben.

d) Die unter c) erhaltene Reaktionslösung wird zu der unter b) vorbereiteten Suspension gegeben und 5 Stunden gerührt. Die Suspension wird eingeengt. Das erhaltene Harz wird in 200 ml Essigester aufgenommen und mit 3N Salzsäure extrahiert. Die Essigesterphase wird mit 3N Natronlauge versetzt und nach Zugabe von Eiswasser wird die wässrige Phase abgetrennt. Die Essigesterphase wird nochmals mit 3N Natronlauge durchmischt, mit Eiswasser verdünnt und extrahiert. Die vereinigten wässrigen Phasen werden auf 0° gekühlt und mit 25-proz. Salzsäure auf pH 1 gestellt. Die entstandene Suspension wird mit Essigester extrahiert. Die vereinigten organischen Phasen werden getrocknet und filtriert. Der Filterkuchen wird mit Essigester gewaschen. Die vereinigten Filtrate werden eingeengt. Das Produkt wird in Eiswasser suspendiert, gerührt und filtriert. Der Filterkuchen wird mit Wasser gewaschen und die Kristalle werden getrocknet. Man erhält 4,63 g (57,6%) (R)-5,6-Dihydro-6-undecyl-2H-pyran-2,4 (3H)-dion, Smp. 84,1-84,8°, das Produkt von Beispiel 3e).

Beispiel 5

a) Unter Stickstoff und Rühren werden 350,1 g 30-proz. Natriummethylatlösung in Methanol mit 550 ml Methanol verdünnt. 264,2 g Malonsäuredimethylester werden zugetropft. Nach Erwärmen der Suspension auf 40° werden 321,0 g 1-Bromhexan zugetropft. Nach 1 Stunde Rühren bei 40°, 2 Stunden unter Rückfluss, 2,5 Stunden bei 65-69° und Abkühlen der Suspension auf Raumtemperatur wird Wasser zugegeben und gerührt. Die organische Phase wird abgetrennt. Die wässrige Phase wird mit Methylenchlorid extrahiert, die vereinigten organischen Extrakte werden getrocknet und filtriert, der Filterkuchen wird mit Methylenchlorid gewaschen und die vereinigten Filtrate werden eingeengt. Nach Destillation des Produktes erhält man 329,3 g (78,3%) n-Hexylmalonsäuredimethylester.

b) Unter Stickstoff und Rühren wird zu 129,78 g des Esters von a) eine Lösung von 40,1 g KOH in 150 ml Methanol zugetropft. Das Reaktionsgemisch wird 2 Stunden gerührt, dann auf Eiswasser gegossen und mit Methylenchlorid extrahiert. Die wässrige Phase wird durch Zugabe von 3N Salzsäure auf pH 2 gestellt und mit Methylenchlorid extrahiert. Die organischen Phasen werden getrocknet und filtriert, der Filterrückstand mit Methylenchlorid gewaschen und die vereinigten Filtrate werden eingeengt. Man erhält 113,9 g (94%) n-Hexylmalonsäuremonomethylester.

c) Unter Stickstoff und Rühren werden einer Suspension von 5,11 g Magnesiumchlorid in 50 ml THF bei 0° eine Lösung von 21,57 g des Esters von b) in 70 ml THF und anschliessend 10,7 g Triäthylamin zugetropft und die entstandene Suspension von n-Hexylmalonsäuremonomethylester-Magnesiumsalz 75 Minuten bei 0° gerührt.

d) Unter Stickstoff und Rühren werden einer Lösung von 7,33 g (R)-3-Hydroxytetradecansäure (Beispiel 4a) in 60 ml THF 6,81 g 1,1'-Carbonyldiimidazol zugegeben. Nach Rühren wird die Reaktionslösung zu der Suspension von n-Hexylmalonsäuremonomethylester-Magnesiumsalz gegeben und 22 Stunden bei Raumtemperatur gerührt. Die Suspension wird eingeengt, wobei 60,35 g Harz zurückbleiben. Dieses wird in 200 ml Essigester aufgenommen, mit 200 ml 3N Salzsäure und mit 600 ml 5-proz. $NaHCO_3$-Lösung extrahiert. Die Essigesterphase wird abgetrennt und unter Rühren bei 10-15° mit 100 ml 25-proz. Salzsäure versetzt. Das Gemisch wird 1,5 Stunden bei 25° gerührt. Die entstandene, homogene Phase wird 16 Stunden bei Raumtemperatur stehen gelassen. Die Suspension wird 4 Stunden bei -20° gelagert, filtriert, der Nutschkuchen mit Wasser gewaschen und die Kristalle getrocknet. Man erhält 3,63 g (34,3%) (R)-5,6-Dihydro-3-hexyl-4-hydroxy-6-undecyl-2H-pyran-2-on, Smp. 104,8-106,2°, das Produkt von Beispiel 3f).

Beispiel 6

a) Unter Stickstoff werden zu 720 g 30%iger Natriummethylatlösung in 1200 ml Methanol 465 g Acetessigsäuremethylester und dann 458 g Aethylbromid zugegeben. Das Reaktionsgemisch wird anschliessend am Rückfluss gekocht. Nach Abdestillation des Methanols wird der Rückstand auf Eiswasser gegossen. Dann wird mit n-Hexan und Wasser extrahiert. Die organischen Phasen werden vereinigt und getrocknet. Nach Abdampfen des Lösungsmittels und Destillation erhält man 328 g (56,9%) 2-Acetylbuttersäuremethylester, Sdp. 77-79°/15 Torr.

b) Unter Argon werden 144,17 g des Methylesters von a) bei 0-5° zu einer Suspension von 26,4 g Natriumhydrid in 1250 ml THF gegeben. Nach 1,5 Stunden Rühren bei 0-5° wird auf -10° abgekühlt. Bei dieser Temperatur werden 675 ml 1,56M Butyllithium in Hexan zugegeben. Nach 30 Minuten Rühren bei -10° wird eine Lösung von 149,3 g Stearinsäuremethylester in 250 ml THF zugetropft. Nach 1,5 Stunden Rühren bei -10° wird die Reaktionslösung unter Argon zu 250 ml 37% Salzsäure und 300 g Eis gegeben. Es wird mit Hexan und Wasser extrahiert. Die vereinigten organischen Phasen werden getrocknet, filtriert und eingedampft.

Der Rückstand wird in 2500 ml THF gelöst, mit 76,1 g DBU versetzt und unter Argon am Rückfluss gekocht. Die abgekühlte Reaktionslösung wird mit 37% Salzsäure und dann mit gesättigter Natriumchloridlösung extrahiert. Die vereinigten organischen Phasen werden getrocknet und eingedampft. Das Produkt wird in Essigsäureäthylester gelöst. Die Lösung wird auf Raumtemperatur abgekühlt und über Nacht bei 25° gerührt. Das Kristallisat wird abgenutscht, mit Essigsäureäthylester gewaschen und getrocknet. Es resultieren 122,5 g (64,7%) 3-Aethyl-6-heptadecyl-4-hydroxy-2H-pyran-2-on, Smp. 101-102°.

c) Zu 100 g des Pyrons von b) werden 100 g Raney-Nickel und 2000 ml THF gegeben. Nach 3 Tagen Hydrieren bei 25° wird der Katalysator abgenutscht und mit THF gewaschen. Das Filtrat wird zur Trockene eingedampft. Der Rückstand wird in Essigsäureäthylester gelöst und bei 10° während 17 Stunden gerührt. Das Kristallisat wird abgenutscht, mit -10° kaltem Essigsäureäthylester gewaschen und 17 Stunden bei 40° getrocknet. Es resultieren 90,54 g (89,6%) rac-(2RS,3RS, 5SR)-2-Aethyl-5-heptadecyl -3-hydroxy-δ-valeriolacton, Smp. 101-102°.

d) Einer Suspension von 191,3 g des δ-Lactons von c) in 1250 ml Toluol werden 138,5 g Benzoesäureanhydrid und anschliessend 2,5 ml Perchlorsäure 70% zugegeben. Nach 2,5 Stunden Rühren wird das Reaktionsgemisch in Toluol mit 1N Natronlauge in 20% Natriumchlorid-Lösung und dann mit gesättigter Natriumchloridlösung extrahiert. Die organischen Phasen werden vereinigt, getrocknet und eingedampft. Es resultieren 243,4 g (100,0%) rac-(2RS,3RS,5SR)-3-Benzoyloxy-2-äthyl -5-heptadecyl-δ-valeriolacton, Smp. 64,5-66°.

e) Unter Argon werden 243 g des Benzoats von d) in 450 ml Toluol bei 40° gelöst. Es werden 1000 ml Methanol und danach 2,5 ml konz. Schwefelsäure zugegeben und das Reaktionsgemisch wird während 20 Stunden bei 25° gerührt. Nach der Neutralisation der Schwefelsäure mit Triäthylamin wird das Lösungsmittel abgedampft. Der Rückstand wird in t-Butylmethyläther gelöst und mit Wasser gewaschen. Die wässrige Phase wird mit t-Butylmethyläther extrahiert und die organischen Phasen werden vereinigt und über Natriumsulfat getrocknet, das Trocknungsmittel wird abgenutscht und mit t-Butylmethyläther gewaschen und anschliessend eingedampft. Es resultieren 257 g (99,1%) rac-(2RS,3RS,5SR)-3-Benzoyloxy-2-äthyl -5-hydroxydocosansäure-methylester.

f) Unter Argon werden 257 g des Hydroxyesters von e) in 1250 ml n-Hexan mit 152 g Benzyl-2,2,2-trichloracetimidat versetzt. Dann werden 3,2 ml Trifluormethansulfonsäure zugegeben. Nach 18 Stunden Rühren bei Raumtemperatur wird der Niederschlag abgenutscht und mit n-Hexan gewaschen. Das Filtrat wird mit 5%iger Natriumbicarbonatlösung und Wasser extrahiert. Die vereinigten Hexanphasen werden getrocknet, filtriert und eingeengt. Nach 20 Stunden Rühren bei -20° wird das Kristallisat abgenutscht, mit n-Hexan gewaschen und verworfen. Das Filtrat wird abgedampft. Es resultieren 239,6 g (78,7%) rac-(2RS,3RS,5SR)-3-Benzoyloxy-5-benzyloxy -2-äthyldocosansäuremethylester, welche ohne Reinigung in der nächsten Stufe eingesetzt werden.

g) Unter Argon werden 239,6 g des Benzyläthers von f) mit einer Lösung von 140 g Kaliumhydroxid in 1250 ml 95% (v/v) Methanol/Wasser versetzt und während 17 Stunden bei 40° gerührt. Anschliessend wird bei 40° eingeengt. Die Suspension wird in t-Butylmethyläther aufgenommen und der Reihe nach mit 10%iger Natriumchloridlösung, 1N Salzsäure und nochmals mit 10%iger Natriumchloridlösung gewaschen. Die organische Phase wird mit Natriumsulfat getrocknet, das Trocknungsmittel abgenuscht und mit t-Butylmethyläther gewaschen. Das Filtrat wird eingedampft. Es resultieren 182,1 g (74,2%) rac-(2RS,3RS,5SR)-5-Benzyloxy-2-äthyl-3-hydroxydocosansäure.

h) Einer Lösung von 182,1 g der β-Hydroxysäure von g) in 1250 ml Essigsäuremethylester werden 33,3 g (S)-(-)-α-Methylbenzylamin zugetropft. Die Lösung wird mit 50 mg Phenäthylaminsalz der (2S,3S,5R)-5-Benzyloxy-2-äthyl-3-hydroxydocosansäure angeimpft und während 20 Stunden stehengelassen. Das Kristallisat wird abgenutscht, mit -20° kaltem Essigsäuremethylester gewaschen und dann getrocknet. Dieses 1. Kristallisat wird in Essigsäuremethylester heiss gelöst, auf 45° abgekühlt und mit 50 mg Phenäthylaminsalz der (2S,3S,5R)-5-Benzyloxy-2-äthyl-3-hydroxydocosansäure angeimpft. Die Lösung wird 20 Stunden bei Raumtemperatur stehengelassen. Das Kristallisat wird abgenutscht, mit -20° kaltem Essigsäuremethylester gewaschen und getrocknet. Die gleiche Arbeitsweise wie mit dem 1. Kristallisat wird mit dem 2. Kristallisat wiederholt. Es resultieren 39,4 g (12,9%) Phenäthylaminsalz der (2S,3S,5R)-5-Benzyloxy-2-äthyl-3-hydroxydocosansäure, Smp. 92-95°.

i) Es werden 39,4 g des Phenäthylaminsalzes aus h) mit 400 ml t-Butylmethyläther und 80 ml 1N Salzsäure versetzt und unter Rühren gelöst. Die organische Phase wird mit Wasser gewaschen, getrocknet, filtriert und eingeengt. Es resultieren 31,4 g (99,4%; 12,8% bezogen auf das δ-Lacton von c) (2S,3S,5R)-5-Benzyloxy-2-äthyl-3-hydroxydocosansäure, Smp. 62-63,5°.

j) Unter Argon werden einer Lösung von 24,5 g der β-Hydroxysäure von i) in 250 ml Pyridin bei 0° 17,6 g Benzolsulfochlorid zugetropft. Nach 20 Stunden Rühren bei 0° werden der Lösung 5 ml Wasser zugetropft. Es wird 1 Stunde bei Raumtemperatur gerührt. Das Pyridin wird abgedampft. Der Kristallbrei wird in t-Butylmethyläther aufgenommen und nacheinander mit 2N Salzsäure, 5%iger Natriumbicarbonatlösung und 10%iger Natriumchloridlösung gewaschen. Die organische Phase wird über Natriumsulfat getrocknet und danach mit Aktivkohle verrührt. Trocknungsmittel und Aktivkohle werden abgenutscht und das Filtrat eingedampft. Es resultieren 23,4 g (99%) (3S,4S)-4-[(R)-2-Benzyloxynonadecyl]-3-äthyl-2-oxetanon.

k) Eine Lösung von 23,4 g des Oxetanons von j) in 250 ml THF wird mit 2,3 g Pd/C 10% versetzt. Nach 5 Stunden Hydrierung wird die Hydrierlösung abgenutscht. Nach Waschen mit THF wird das Filtrat eingedampft, der Rückstand in n-Hexan gelöst und mit (3S,4S)-3-Aethyl-4-[(R)-2-hydroxynonadecenyl]-2-oxetanon angeimpft. Nach 18 Stunden wird das Kristallisat abgenutscht, mit Hexan gewaschen und getrocknet. Es resultieren 16,1 g (84,1%) (3S,4S)-3-Aethyl-4-[(R)-2-hydroxynonadecyl]-2-oxetanon, Smp. 66,5-68°.

Beispiel 7

Unter Argon werden unter Rühren 19,13 g des Hydroxy-β-lactons von Beispiel 6k), 10,34 g N-Formyl-(S)-leucin und 19,70 g Triphenylphosphin in 400 ml THF gelöst. Es wird auf 0° abgekühlt und eine Lösung von 14,5 g Azodicarbonsäurediäthylester in 50 ml THF zugetropft. Die Reaktionslösung wird 4 Stunden bei 0° gerührt. Anschliessend wird das Lösungsmittel abgedampft. Der Kristallbrei wird zwischen Hexan und 70% Methanol/Wasser mehrmals verteilt. Die vereinigten Hexan-Phasen werden über Natriumsulfat getrocknet, das Trocknungsmittel wird abgenutscht und mit Hexan gewaschen. Nach Abdestillieren des Hexans wird das Produkt in Hexan gelöst und nach 20 Stunden wird das Kristallisat abgenutscht, mit Hexan gewaschen und getrocknet. Es resultieren 20,74 g (79,2%) N-Formyl-L-leucin (S)-1-[[(2S,3S)-3-äthyl-4-oxo-2-oxetanyl]methyl]octadecylester, Smp. 61-62°.

Beispiel 8

a) Unter Argon werden unter Rühren einer Suspension von 177.3 g (2S,3S,5R)-2-Hexyl-3-hydroxy-5-undecyl-δ-valeriolacton in 1250 ml Toluol 135,7 g Benzoesäureanhydrid und nach 10 Minuten Rühren bei Raumtemperatur 2,5 ml 70%ige Perchlorsäure zugegeben. Nach 4 Stunden Rühren wird die Reaktionslösung in Toluol mit 1N Natronlauge und Wasser extrahiert. Die vereinigten Toluol-Phasen werden über Natriumsulfat getrocknet, das Trocknungsmittel wird abgenutscht und mit Toluol gewaschen. Abdampfen des Lösungsmittels ergibt 249,9 g (109%) (2S,3S,5R)-3-Benzoyloxy-2-hexyl-5-undecyl-δ-valeriolacton, welches ohne Reinigung in der nächsten Stufe eingesetzt wird.

b) Unter Argon werden 249,8 g des Benzoats von a) in 1250 ml Methanol unter Rühren mit 2,5 ml konz. Schwefelsäure versetzt und während 18 Stunden bei 35° gerührt. Anschliessend wird der pH-Wert der Reaktionslösung mit Triäthylamin auf 9 eingestellt und das Methanol abgedampft. Der Rückstand wird in Hexan aufgenommen, mit Wasser gewaschen und die Wasserphase mit Hexan extrahiert. Nach dem Trocknen der vereinigten Hexanphasen wird filtriert und das Filtrat wird vom Hexan befreit. Es resultieren 246,5 g (100,5%) (2S,3S,5R)-3-Benzoyloxy-2-hexyl -5-hydroxyhexadecansäure-methylester, welcher ohne Reinigung in der nächsten Stufe eingesetzt wird.

c) Unter Argon werden 246,5 g des Hydroxyesters von b) in 1250 ml Hexan unter Rühren mit 152 g Benzyl-2,2,2-trichloracetimidat versetzt. Dann werden 3,2 ml Trifluormethansulfonsäure zugegeben. Nach 17 Stunden Rühren bei Raumtemperatur wird der Niederschlag abgenutscht und mit Hexan gewaschen. Das Filtrat wird mit 5%iger Natriumbicarbonatlösung und dann mit Wasser extrahiert. Die vereinigten Hexanphasen werden getrocknet, filtriert und eingedampft. Es resultieren 308 g (106,2%) (2S,3S,5R)-3-Benzoyloxy-5-benzyloxy -2-hexylhexadecansäuremethylester, welcher ohne Reinigung in der nächsten Stufe eingesetzt wird.

d) Unter Argon werden 308,5 g des Benzyläthers von c) in 1125 ml Methanol mit einer Lösung von 140 g Kaliumhydroxid in 125 ml Wasser versetzt. Das Reaktionsgemisch wird bei 40° während 17 Stunden gerührt und anschliessend eingeengt. Die Suspension wird in Hexan aufgenommen und der Reihe nach mit 10%iger Natriumchloridlösung, 1N Salzsäure und 10%iger Natriumchloridlösung extrahiert. Die organische Phase wird mit Natriumsulfat getrocknet, das Trocknungsmittel wird abgenutscht und mit Hexan gewaschen. Das Filtrat wird auf 1000 ml eingeengt und bei -20° gerührt. Das Kristallisat wird abgenutscht, mit Hexan gewaschen und verworfen. Das Filtrat ergibt nach Abdampfen des Lösungsmittels ein Produkt, das in Essigsäuremethylester gelöst und unter Rühren mit Benzylamin versetzt wird. Die Lösung wird mit dem Benzylaminsalz der (2S,3S,5R)-5-Benzyloxy-2-hexyl-3-hydroxyhexadecansäure angeimpft und anschliessend auf -5° abgekühlt. Dann wird 17 Stunden bei -10° kristallisiert. Das Kristallisat wird abgenutscht, mit Essigsäuremethylester gewaschen und danach getrocknet. Es resultieren 116,7 g (41% bezogen auf das Ausgangsvaleriolacton von a) Benzylaminsalz der (2S,3S,5R)-5-Benzyloxy-2-hexyl-3-hydroxyhexadecansäure, Smp. 66-68°.

e) Es werden 116,7 g Benzylaminsalz von d) mit 1000 ml Hexan und 250 ml 1N Salzsäure unter Rühren versetzt. Die organische Phase wird mit Wasser gewaschen, getrocknet und eingedampft. Man erhält 95,4 g (100,6%; 41,2% bezogen auf das Ausgangsvaleriolacton von a) (2S,3S,5R)-5-Benzyloxy-2-hexyl-3-hydroxyhexadecansäure, des Produkts von Beispiel 1i).

Beispiel 9

a) Unter Argon und unter Rühren werden 110,2 g 2-Acetyloctansäuremethylester (das Produkt von Beispiel la) bei 0-5° zu einer Suspension von 14,4 g Natriumhydrid 97% in 750 ml THF getropft. Es wird 1 Stunde bei Raumtemperatur gerührt und anschliessend auf -12° abgekühlt. 370 ml 1,56M Butyllithium in Hexan werden innert 1 Stunde bei -12 bis -10° zugegeben. Es wird 1 Stunde bei -12° weitergerührt. Zur erhaltenen Lösung werden 92,2 g Laurinaldehyd bei -10° zugetropft. Es wird 1 Stunde bei dieser Temperatur nachgerührt. Die Reaktionslösung wird innert 5 Minuten unter Rühren zu 600 ml Wasser gegeben. Es wird 1 Stunde bei Raumtemperatur weitergerührt und anschliessend mit 100 ml 37% Salzsäure neutralisiert. Nach dem Abtrennen der wässrigen Phase wird mit 300 ml gesättigter Natriumchloridlösung gewaschen, die organische Phase über Natriumsulfat getrocknet und das Trocknungsmittel abgenutscht. Nach Abdampfen des Lösungsmittels wird das Produkt mit Hexan verrührt. Das Kristallisat wird abgenutscht, mit Hexan gewaschen und getrocknet. Es resultieren 130,61 g (74.1%) rac-5,6-Dihydro-3-hexyl-4-hydroxy-6-undecyl-2H-pyran-2-on, Smp. 121,5-122,5°.

b) Unter Rühren werden zu 50 g des Dihydropyrons von a) 1000 ml Essigsäureäthylester und 12,5 g Raney-Nickel gegeben. Nach 17 Stunden Hydrierung unter Rühren bei 30° wird der Katalysator abgenutscht und mit Essigsäureäthylester gewaschen. Das Filtrat wird eingeengt und bei -10° gerührt. Das Kristallisat wird abgenutscht, mit Essigsäureäthylester gewaschen und getrocknet. Es resultieren 45,4 g (90,3%) rac-(2RS,3RS,5SR)-2-Hexyl-3-hydroxy -5-undecyl-δ-valeriolacton, Smp. 98,5-99,5°, das Produkt von Beispiel 1c).

Beispiel 10

a) Einer Lösung von 117 g Meldrumsäure und 131 ml Pyridin in 1,5 l Methylenchlorid werden 270 ml Stearinsäurechlorid bei maximal 15°C zugetropft. Nach Rühren wird das Reaktionsgemisch mit 4N Salzsäure gewaschen, die wässrige Phase mit Methylenchlorid nachextrahiert, die Methylenchloridphase getrocknet und eingeengt. Der Rückstand wird in Methanol aufgenommen und unter Rückfluss gerührt. Nach dem Abkühlen werden die ausgefallenen Kristalle abfiltriert, in Methylenchlorid gelöst und an Kieselgel mit Methylenchlorid chromatographiert. Man erhält 175 g Methyl-3-oxoeicosanoat, Smp. 52-54°C.

b) Einer Lösung von 9,1 mg [(R)-2,2'-Bis(diphenylphosphino) -6,6'-dimethylbiphenyl]ruthenium-diacetat in 20 ml Methylenchlorid werden 1,84 mg Acetylchlorid in 1,84 ml Methanol zugegeben. Die erhaltene Lösung wird zusammen mit 39,8 g des Ketoesters von a) und 170 ml Methanol bei 35 bar Wasserstoff und 60°C hydriert. Nach Zusatz von Methylenchlorid wird zur Trockene eingedampft. Chromatographie an Kieselgel mit Aether und Umkristallisation aus n-Hexan liefert 35,7 g (R)-3-Hydroxyeicosansäuremethylester, Smp. 64-64,5°C.

c) Analog Beispiel 4 wird aus dem (R)-3-Hydroxyeicosansäuremethylester über die (R)-3-Hydroxyeicosansäure vom Smp. 89° das (R)-5,6-Dihydro-6-heptadecyl-2H-pyran-2,4(3H)-dion vom Smp. 97° hergestellt.

Analog Beispiel 3f) und g) wird das obige Pyrandion mit Acetaldehyd über das (R)-3-Aethyl-5,6-dihydro-6-heptadecyl-4-hydroxy-2H-pyran-2-on vom Smp. 110,5-112,5° in das 2S,3S,5R-2-Aethyl-5-heptadecyl-3-hydroxy-δ-valeriolacton, $[\alpha]_D^{20}$ = -39,8° (c = 1 in $CHCl_3$) übergeführt.
Analog Beispiel 2h) bis k) kann man das obige Pyranon via (3S,4S,6R)-4-(t-Butyldimethylsiloxy)-3-äthyl -3,4,5,6-tetrahydro-6-heptadecyl-2H-pyran-2-on,

(2S,3S,5R)-3-(t-Butyldimethylsiloxy)-2-äthyl-5-hydroxydocosansäure-benzylester und

(2S,3S,5R)-5-Benzyloxy-3-(t-butyldimethylsiloxy)-2-äthyldocosansäurebenzylester

in die (2S,3S,5R)-5-Benzyloxy-3-hydroxy-2-äthyldocosansäuce (das Produkt des Beispiels 6h) als Benzylaminsalz überführen.

Beispiel 11

a) Analog Beispiel 1b) wird der 2-Acetyloctansäuremethylester (Beispiel 1a) mit Methylhexanoat in das 3-Hexyl-4-hydroxy-6-pentyl-2H-pyran-2-on, Smp. 110,8-111,7° übergeführt.

b) Analog Beispiel 9a) wird der 2-Acetyloctansäuremethylester mit Hexanal in das rac-5,6-Dihydro-3-hexyl-4-hydroxy-6-pentyl-2H-pyran-2-on, Smp. 137-139° übergeführt.

c) Hydrierung des Pyranons von a) oder b) führt analog Beispiel 1c) bzw. 9c) zum rac-(2RS,3RS,5SR)-2-Hexyl-3-hydroxy -5-pentyl-δ-valeriolacton, Smp. 117-118°.

d) Analog Beispiel 1d) bis k) wird das Lacton von Beispiel 11c) via rac-(2RS,3RS,5SR)-3-Benzoyloxy-2-hexyl -5-pentyl-δ-valeriolacton,

rac-(2RS,3RS,5SR)-3-Benzoyloxy-2-hexyl-5-hydroxydecansäuremethylester,

rac-(2RS,3RS,5SR)-3-Benzoyloxy-5-benzyloxy-2-hexyldecansäuremethylester,

rac-(2RS,3RS,5SR)-5-Benzyloxy-2-hexyl-3-hydroxydecansäuremethylester,

(2S,3S,5R)-5-Benzyloxy-2-hexyl-3-hydroxydecansäure-(S)-α-methylbenzylaminsalz, Smp. 116-117°,

(2S,3S,5R)-5-Benzyloxy-2-hexyl-3-hydroxydecansäure, $[\alpha]_D^{20}$ = -31,5° (c = 0,635 in $CHCl_3$) und

(3S,4S)-4-[(R)-2-Benzyloxyheptyl]-3-hexyl-2-oxetanon, $[\alpha]_D^{20}$ = 63,1° (c = 1 in $CHCl_3$),

in das (3S,4S)-3-Hexyl-4-[(R)-2-hydroxyheptyl]-2-oxetanon, $[\alpha]_D^{20}$ = -51,9° (c = 1 in $CHCl_3$) übergeführt.

e) Letzteres wird analog Beispiel 2 in den N-Formyl-L-valin-(S)-1-[[(2S,3S)-3-hexyl-4-oxo -2-oxetanyl]methyl]-hexylester (Valilacton) Smp. 57,0-57,3°

bzw. in den N-Formyl-L-leucin-(S)-1-[[(2S,3S)-3-hexyl-4-oxo -2-oxetanyl]methyl]hexylester, Smp. 50-50,5° verestert.

Beispiel 12

a) 3,55 g rac-(2RS,3RS,5SR)-2-Hexyl-3-hydroxy -5-undecyl-δ-valeriolacton (Beispiel 1c), 30 ml TBME, 12,6 mg Pyridinium-p-toluolsulfonat und 2,66 g 95%iges 3,4-Dihydro-2H-pyran werden bei 50° während 20 Stunden gerührt. Die Lösung wird mit wässriger NaCl-Lösung gewaschen und dann getrocknet. Abdampfen des Lösungsmittels und Trocknen ergeben 4,41 g (100,5%) (2RS,3RS,SSR)-2-Hexyl-3-(tetrahydro-2H-pyran-2-yloxy) -5-undecyl-δ-valeriolacton, Smp. 39-41°.

b) Eine Lösung von 4,41 g des Aethers von a) in 30 ml t-Butylmethyläther wird mit 10 ml 2N Natronlauge versetzt und während 20 Stunden gerührt. Nach Abtrennen der wässrigen Phase wird mit 10 ml 10%iger, wässriger NaCl-Lösung gewaschen und die organische Phase bei 55° eingedampft. Der Rückstand wird in 30 ml TBME gelöst und das Lösungsmittel erneut abgedampft. Eine Lösung des Rückstands in 30 ml THF wird zuerst eingedampft und dann getrocknet. Es resultieren 4,81 g (100,5%) des Natriumsalzes der (2RS,3RS,5SR)-2-Hexyl-5-hydroxy-3-(tetrahydro-2H-pyran -2-yloxy)hexadecansäure.

c) 4,80 g des Natriumsalzes von b) werden unter Argon mit 50 ml THF, 2,57 g Benzylbromid und 0,495 g Natriumhydrid versetzt und bei 50° während 24 Stunden gerührt. Die Suspension wird mit 10 ml 2N Salzsäure auf pH 0 hydrolysiert, 2 Stunden bei 50° gerührt und dann die wässrige Phase abgetrennt. Nach Waschen mit wässriger NaCl-Lösung wird die organische Phase eingedampft. Es resultieren 5,42 g rac-(2RS,3RS,5SR)-5-Benzyloxy-2-hexyl-3-hydroxyhexadecansäure, Produkt des Beispiels 1g), welche wie im Beispiel 1h) in ihre Antipoden gespalten wird.

Beispiel 13

a) Unter Argon werden 177,3 mg rac-(2RS,3RS,5SR)-2-Hexyl-3-hydroxy-5-undecyl-δ-valeriolacton (Beispiel 1c), 750 ml TBME, 86,7 g 97%iges 3,4-Dihydro-2H-pyran und 0,314 g Pyridinium-p-toluolsulfonat 20 Stunden bei 50° gerührt. Zur Reaktionslösung werden 500 ml 2N Natronlauge zugegeben. Nach 2,5 Stunden Rühren bei 50° wird die wässrige Phase abgetrennt und die organische Phase mit 500 ml 10%iger Natriumchloridlösung gewaschen.

b) Nach 24 Stunden Kochen am Wasserabscheider wird der Natriumsalz-Brei abgekühlt, unter Argon nacheinander mit 152,7 g Benzylbromid und 99,1 g Na-t-Butylat versetzt und 24 Stunden bei Raumtemperatur gerührt. Nach Zugabe von 750 ml 2N Salzsäure und 22 Stunden Rühren bei 50° wird abgekühlt, die wässrige Phase abgetrennt und die organische Phase mit Natriumchloridlösung gewaschen, getrocknet und filtriert. Nach Abdampfen des Lösungsmittels erhält man 349,8 g rac-(2RS,3RS,5SR)-5-Benzyloxy-2-hexyl-3-hydroxydecansäure (Produkt der Beispiele 1g und 12c).

c) In einer Variante zu b) wird das Produkt von a) 17 Stunden am Wasserabscheider gekocht. Dann wird das Lösungsmittel abdestilliert. Nach Trocknen erhält man 253,4 g des Natriumsalzes der (2RS,3RS,5SR)-2-Hexyl-5-hydroxy-3-(tetrahydro-2H-pyran-2-yloxy)hexadecansäure.

d) Eine Lösung des Produkts von c) in 2 l THF wird einer Suspension von 24,7 g 97%igem NaH in 500 ml THF und 131 g Benzylbromid bei 50° unter Rühren gegeben. Nach 22 Stunden Rühren werden 500 ml 2N Salzsäure zugegeben. Nach Abkühlen wird die wässrige Phase abgetrennt und mit Natriumchloridlösung gewaschen. Die erhaltene Lösung der racemischen Hydroxysäure, rac-(2RS,3RS,5SR)-5-Benzyloxy-2-hexyl-3-hydroxydecansäure (Beispiel 13b) wird zur Racematspaltung (Beispiel 1h) verwendet.

**Patentansprüche**

**Patentansprüche für folgende Vertragsstaaten : AT, BE, CH, DE, DK, FR, GB, GR, IT, LI, LU, NL, SE**

**1.** Verfahren zur Herstellung von Oxetanonen der Formel

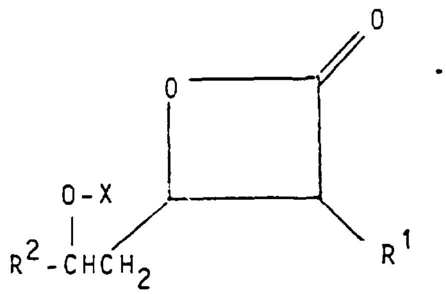

I

worin

| | |
|---|---|
| $R^1$ und $R^2$ | in einer anderen als der α- oder β-Stellung gegebenenfalls durch ein O-Atom unterbrochenes Alkyl mit bis zu 17 C-Atomen; oder gegebenenfalls durch 1 bis 3 $C_{1-6}$-Alkyl- oder $C_{1-6}$-Alkoxygruppen ringsubstituiertes Benzyl, |
| X | Wasserstoff oder eine Gruppe der Formel $(R^3,R^4)NCH(R^5)(CH_2)_n$-CO- |
| $R^3$ | Wasserstoff, $C_{1-3}$-Alkyl oder $C_{1-3}$-Alkanoyl, |
| $R^4$ | Wasserstoff oder $C_{1-3}$-Alkyl, und |
| $R^5$ | Wasserstoff, eine Gruppe Ar oder Ar-$C_{1-3}$-Alkyl oder gegebenenfalls durch Y unterbrochenes und gegebenenfalls durch Z substituiertes $C_{1-7}$-Alkyl sind, oder |
| $R^4$ mit $R^5$ | zusammen mit dem N-Atom, an dem sie gebunden sind, einen 4- bis 6-gliedrigen gesättigten Ring bildet, |
| Y | Sauerstoff, Schwefel oder eine Gruppe $N(R^6)$, $C(O)N(R^6)$ oder $N(R^6)C(O)$, |
| Z | eine Gruppe -(O oder S)-$R^7$, -$N(R^7,R^8)$, -$C(O)N(R^7,R^8)$ oder -$N(R^7)C(O)R^8$, |
| n | die Zahl 1 oder 0 ist, wobei falls n die Zahl 1 ist, $R^5$ Wasserstoff ist, |
| Ar | durch 1 bis 3 Gruppen $R^9$ oder $OR^9$ substituiertes Phenyl, und |
| $R^6$ bis $R^9$ | Wasserstoff oder $C_{1-3}$-Alkyl sind. |

und von Salzen der Oxetanone der Formel I, worin X nicht Wasserstoff ist, mit schwachen Säuren, dadurch gekennzeichnet, dass man

a) ein β-Hydroxy-δ-lacton der Formel

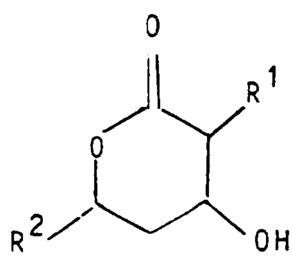

II

veräthert,

b) den erhaltenen Aether der Formel

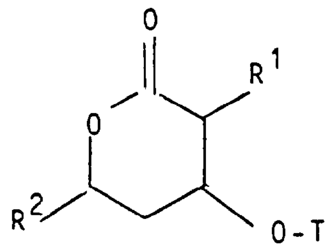

III

worin T eine leicht spaltbare Aethergruppe ist, mit einer Base öffnet,

c) das erhaltene Salz der Formel

$$R^2\text{-CHOHCH}_2\text{CH(O-T)CH(R}^1\text{)COO-M} \qquad \text{IV}$$

worin M ein Alkali- oder Erdalkalimetall ist, in beliebiger Reihenfolge mit einem Arylmethylhalogenid und einer Base umsetzt und

d) den entstandenen Diäther der Formel

$$R^2\text{-CH(OCH}_2\text{-Ar)CH}_2\text{CH(O-T)CH(R}^1\text{)COO-M} \qquad \text{V}$$

mit einer Säure selektiv spaltet,

e) die erhaltene β-Hydroxysäure der Formel

$$R^2\text{-CH(OCH}_2\text{-Ar)CH}_2\text{CHOHCH(R}^1\text{)COOH} \qquad \text{VI}$$

gegebenenfalls nach Aufspaltung in ihre Enantiomeren cyclisiert,

f) den erhaltenen β-Lactonäther der Formel

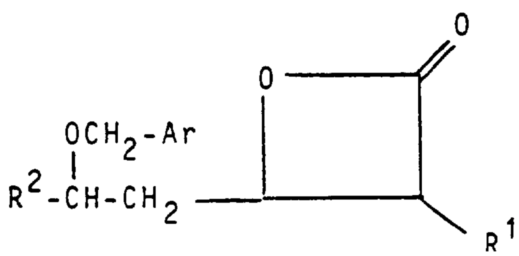

VII

spaltet, und

g) den erhaltenen β-Lactonalkohol der Formel I, worin X Wasserstoff ist, gegebenenfalls mit einem die Gruppe X einführenden Mittel verestert und

h) den erhaltenen Ester, gegebenenfalls in Form eines Salzes mit einer schwachen Säure, isoliert.

**2.** Verfahren nach Anspruch 1, dadurch gekennzeichnet, dass man in der Stufe c) ein Alkalimetallhydrid als Base verwendet.

**3.** Variante des Verfahrens nach Anspruch 1, dadurch gekennzeichnet, daß man zur Herstellung der β-Hydroxysäure der Formel VI von Anspruch 1:

a) ein Salz der Formel IV von Anspruch 1 mit einem Halogenid der Formel $R^{10}$-Hal, worin $R^{10}$ $C_{1-4}$-Alkyl oder Aryl-$C_{1-4}$ -alkyl ist, verestert,

b) den erhaltenen Ester der Formel

$$R^2\text{-CHOHCH}_2\text{CH(O-T)CH(R}^1\text{)COO-R}^{10} \qquad \text{IV-A}$$

veräthert und

c) den erhaltenen Diäther der Formel

$$R^2\text{-CH(OCH}_2\text{-Ar)CH}_2\text{CH(OT)CH(R}^1\text{)COO-R}^{10} \qquad \text{V-A}$$

in beliebiger Reihenfolge verseift und in β-Stellung spaltet.

**4.** Variante des Verfahrens nach Anspruch 1, dadurch gekennzeichnet, daß man zur Herstellung der β-Hydroxysäure der Formel VI von Anspruch 1:

a) ein β-Hydroxy-δ-lacton der Formel II von Anspruch 1 verestert,

b) den erhaltenen Ester der Formel

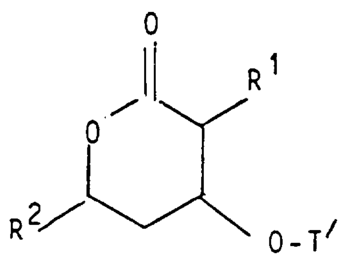

III'

worin T' Aroyl ist,
säurekatalytisch in Gegenwart eines Alkohols der Formel $R^{10}$-OH zu einem Ester der Formel

$$R^2\text{-CHOHCH}_2\text{CH(O-T')CH(R}^1\text{)COO-R}^{10} \qquad \text{IV-B}$$

öffnet,

c) den Ester der Formel IV-B veräthert und

d) den erhaltenen Aetherdiester der Formel

$$R^2\text{-CH(OCH}_2\text{-Ar)CH}_2\text{CH(O-T')CH(R}^1\text{)COO-R}^{10} \qquad \text{V-B}$$

doppelt verseift.

**5.** Verfahren nach Anspruch 1 oder 4, dadurch gekennzeichnet, daß man zur Herstellung der β-Hydroxy-δ-lactone der Formel II von Anspruch 1:

a) einen β-Hydroxyester der Formel

$$R^2\text{-CHOHCH}_2\text{COO-R} \qquad \text{VIII}$$

worin R $C_{1-4}$-Alkyl ist,
verseift,

b) das Imidazolid der erhaltenen β-Hydroxysäure der Formel

$$R^2\text{-CHOHCH}_2\text{COOH} \qquad \text{IX}$$

mit dem Mg-Salz des Malonsäureesterderivats der Formel

$$\text{HOCOCH}(R^1)\text{COO-R} \qquad \text{X}$$

umsetzt,

c) den entstandenen δ-Hydroxy-β-ketoester der Formel

$$R^2\text{-CHOHCH}_2\text{COCH}(R^1)\text{COO-R} \qquad \text{XI}$$

cyclisiert und

d) das erhaltene β-Keto-δ-lacton der Formel

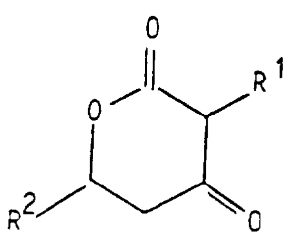

XII

katalytisch hydriert.

**6.** Verfahren nach Anspruch 1 oder 4, dadurch gekennzeichnet, daß man zur Herstellung der β-Hydroxy-δ-lactone der Formel II von Anspruch 1:

a) einen β-Ketoester der Formel

$$CH_3\text{COCH}(R^1)\text{COO-R} \qquad \text{XIII}$$

mit einem Ester der Formel

$$R^2\text{-COO-R} \qquad \text{XIV}$$

umsetzt,

b) den erhaltenen Diketoester der Formel

$R^2$-$COCH_2COCH(R^1)COO$-R XV

cyclisiert und

c) das erhaltene Pyron der Formel

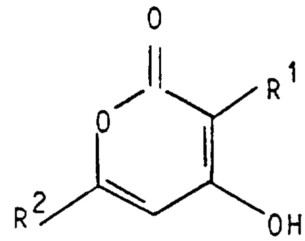

XVI

katalytisch hydriert.

**7.** Verfahren nach Anspruch 1 oder 5, dadurch gekennzeichnet, dass man zur Herstellung der Verbindung der Formel II:

a) einen β-Ketoester der Formel

$CH_3COCH(R^1)COO$-R XIII

mit einem Aldehyd der Formel

$R^2$-CHO XVII

umsetzt,

b) den entstandenen δ-Hydroxy-β-ketoester der Formel

$R^2$-$CHOHCH_2COCH(R^1)COO$-R XI

cyclisiert und

c) das erhaltene β-Keto-δ-lacton der Formel

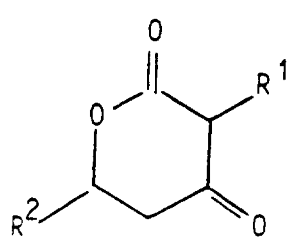

XII

katalytisch hydriert.

**8.** Variante des Verfahrens nach Anspruch 5, dadurch gekennzeichnet, daß man zur Herstellung der β-Keto-δ-lactone der Formel XII von Anspruch 5:

a) einen β-Hydroxyester der obigen Formel

$$R^2\text{-CHOHCH}_2\text{COO-R} \qquad \text{VIII}$$

veräthert,

b) den erhaltenen Aether der Formel

$$R^2\text{-CH(OCH}_2\text{-Ar)CH}_2\text{COO-R} \qquad \text{XVIII}$$

verseift,

c) die erhaltene Aethersäure der Formel

$$R^2\text{-CH(OCH}_2\text{-Ar)CH}_2\text{COOH} \qquad \text{XIX}$$

halogeniert,

d) das erhaltene Säurehalogenid mit Meldrumsäure umsetzt,

e) die erhaltene Verbindung der Formel

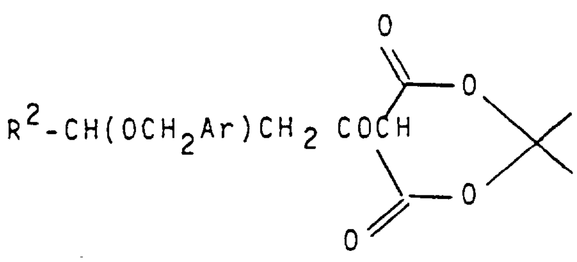

XX

hydrogenolysiert und zu einem β-Keto-δ-lacton der Formel

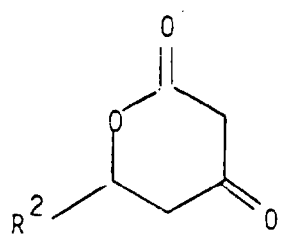

XXI

cyclisiert, und

f) das β-Keto-δ-lacton der obigen Formel XXI mit einem die Gruppe $R^1$ bzw. $-CH_2-R^{11}$ einführenden Aldehyd der Formel

$$R^{11}\text{-CHO} \qquad \text{XXII}$$

worin $R^{11}$ zusammen mit der Methylengruppe die Gruppe $R^1$ darstellt,

zu einem β-Keto-δ-lacton der obigen Formel XII umsetzt.

**9.** Variante des Verfahrens nach Anspruch 8, dadurch gekennzeichnet, daß man zur Herstellung der β-Keto-δ-lactonen der Formel XXI von Anspruch 8:

a) das Imidazolid der β-Hydroxysäure der Formel

$$R^2\text{-CHOHCH}_2\text{COOH} \qquad \text{IX}$$

mit dem Mg-Salz eines Malonsäuremononiederalkylesters umsetzt und

b) den entstandenen δ-Hydroxy-β-ketoester der Formel

$$R^2\text{-CHOHCH}_2\text{COCH}_2\text{COO-R} \qquad \text{XXIII}$$

zum β-Keto-δ-lacton der obigen Formel XXI cyclisiert.

**10.** Verfahren nach Anspruch 1, 2, 5 oder 7, worin $R^1$ n-Hexyl und $R^2$ Undecyl ist.

**11.** Die β-Hydroxy-δ-lactone, β-Keto-δ-lactone und Pyrone der Formeln II, XII bzw. XVI

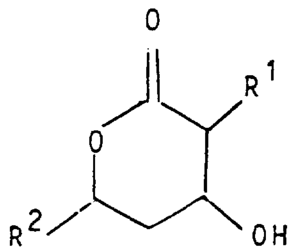 , 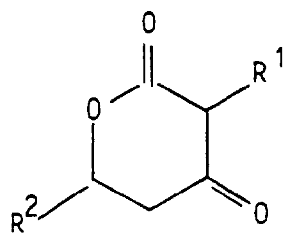 , 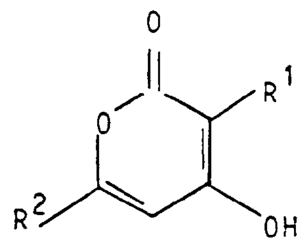

aus der Gruppe der folgenden:

rac-(2RS,3RS,5SR)-2-Hexyl-3-hydroxy-5-undecyl-δ-valeriolacton,
rac-(2RS,3RS,5SR)-2-Aethyl-5-heptadecyl-3-hydroxy-δ-valeriolacton,
(2S,3S,5R)-2-Aethyl-5-heptadecyl-3-hydroxy-δ-valeriolacton und
rac-(2RS,3RS,5SR)-2-Hexyl-3-hydroxy-5-pentyl-δ-valeriolacton;
rac-5,6-Dihydro-3-hexyl-4-hydroxy-6-undecyl-2H-pyran-2-on,
(R)-3-Aethyl-5,6-dihydro-6-heptadecyl-4-hydroxy-2H-pyran-2-on und
rac-5,6-Dihydro-3-hexyl-4-hydroxy-6-pentyl-2H-pyran-2-on;
3-Hexyl-4-hydroxy-6-undecyl-2H-pyran-2-on,
3-Aethyl-6-heptadecyl-4-hydroxy-2H-pyran-2-on und
3-Hexyl-4-hydroxy-6-pentyl-2H-pyran-2-on.

**12.** Die β-Keto-δ-lactone der Formel XXI von Anspruch 8, worin $R^2$ die in Anspruch 1 angegebene Bedeutung hat, die Anzahl C-Atome in der Alkylgruppe $R^2$ jedoch mehr als 9 betragen soll, insbesondere die folgenden:

(R)-5,6-Dihydro-6-undecyl-2H-pyran-2,4(3H)-dion und
(R)-5,6-Dihydro-6-heptdecyl-2H-pyran-2,4(3H)-dion.

**Patentansprüche für folgenden Vertragsstaat : ES**

1. Verfahren zur Herstellung von Oxetanonen der Formel

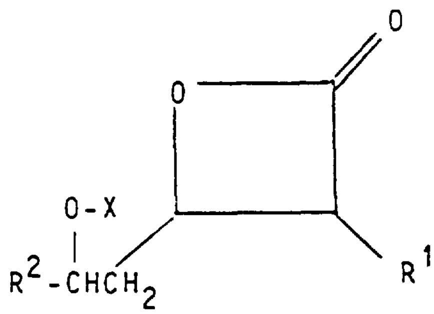

I

worin

| | |
|---|---|
| $R^1$ und $R^2$ | in einer anderen als der α- oder β-Stellung gegebenenfalls durch ein O-Atom unterbrochenes Alkyl mit bis zu 17 C-Atomen; oder gegebenenfalls durch 1 bis 3 $C_{1-6}$-Alkyl- oder $C_{1-6}$-Alkoxygruppen ringsubstituiertes Benzyl, |
| X | Wasserstoff oder eine Gruppe der Formel $(R^3,R^4)NCH(R^5)(CH_2)_n$-CO- |
| $R^3$ | Wasserstoff, $C_{1-3}$-Alkyl oder $C_{1-3}$-Alkanoyl, |
| $R^4$ | Wasserstoff oder $C_{1-3}$-Alkyl, und |
| $R^5$ | Wasserstoff, eine Gruppe Ar oder Ar-$C_{1-3}$-Alkyl oder gegebenenfalls durch Y unterbrochenes und gegebenenfalls durch Z substituiertes $C_{1-7}$-Alkyl sind, oder |
| $R^4$ mit $R^5$ | zusammen mit dem N-Atom, an dem sie gebunden sind, einen 4- bis 6-gliedrigen gesättigten Ring bildet, |
| Y | Sauerstoff, Schwefel oder eine Gruppe $N(R^6)$, $C(O)N(R^6)$ oder $N(R^6)C(O)$, |
| Z | eine Gruppe -(O oder S)-$R^7$, -$N(R^7,R^8)$, -$C(O)N(R^7,R^8)$ oder -$N(R^7)C(O)R^8$, |
| n | die Zahl 1 oder 0 ist, wobei falls n die Zahl 1 ist, $R^5$ Wasserstoff ist, |
| Ar | durch 1 bis 3 Gruppen $R^9$ oder $OR^9$ substituiertes Phenyl, und |
| $R^6$ bis $R^9$ | Wasserstoff oder $C_{1-3}$-Alkyl sind, |

und von Salzen der Oxetanone der Formel I, worin X nicht Wasserstoff ist, mit schwachen Säuren, dadurch gekennzeichnet, dass man

a) ein β-Hydroxy-δ-lacton der Formel

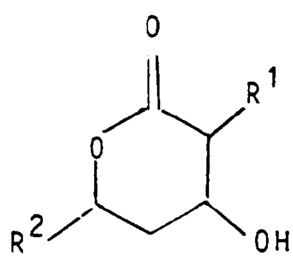

II

veräthert,

b) den erhaltenen Aether der Formel

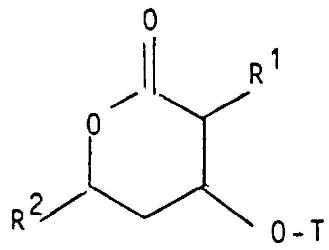

III

worin T eine leicht spaltbare Aethergruppe ist,
mit einer Base öffnet,

c) das erhaltene Salz der Formel

$$R^2\text{-CHOHCH}_2\text{CH(O-T)CH(R}^1\text{)COO-M} \qquad IV$$

worin M ein Alkali- oder Erdalkalimetall ist,
in beliebiger Reihenfolge mit einem Arylmethylhalogenid und einer Base umsetzt und

d) den entstandenen Diäther der Formel

$$R^2\text{-CH(OCH}_2\text{-Ar)CH}_2\text{CH(O-T)CH(R}^1\text{)COO-M} \qquad V$$

mit einer Säure selektiv spaltet,

e) die erhaltene β-Hydroxysäure der Formel

$$R^2\text{-CH(OCH}_2\text{-Ar)CH}_2\text{CHOHCH(R}^1\text{)COOH} \qquad VI$$

gegebenenfalls nach Aufspaltung in ihre Enantiomeren cyclisiert.

f) den erhaltenen β-Lactonäther der Formel

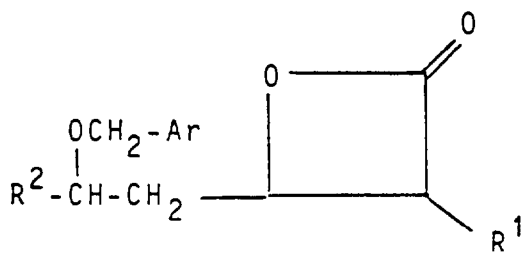

VII

spaltet, und

g) den erhaltenen β-Lactonalkohol der Formel I, worin X Wasserstoff ist, gegebenenfalls mit einem die Gruppe X einführenden Mittel verestert und

h) den erhaltenen Ester, gegebenenfalls in Form eines Salzes mit einer schwachen Säure, isoliert.

**2.** Verfahren nach Anspruch 1, dadurch gekennzeichnet, dass man in der Stufe c) ein Alkalimetallhydrid als Base verwendet.

**3.** Variante des Verfahrens nach Anspruch 1, dadurch gekennzeichnet, daß man zur Herstellung der β-Hydroxysäure der Formel VI von Anspruch 1:

a) ein Salz der Formel IV von Anspruch 1 mit einem Halogenid der Formel $R^{10}$-Hal, worin $R^{10}$ $C_{1-4}$-Alkyl oder Aryl-$C_{1-4}$-alkyl ist, verestert,

b) den erhaltenen Ester der Formel

$$R^2\text{-CHOHCH}_2\text{CH(O-T)CH(R}^1\text{)COO-R}^{10} \qquad \text{IV-A}$$

veräthert und

c) den erhaltenen Diäther der Formel

$$R^2\text{-CH(OCH}_2\text{-Ar)CH}_2\text{CH(OT)CH(R}^1\text{)COO-R}^{10} \qquad \text{V-A}$$

in beliebiger Reihenfolge verseift und in β-Stellung spaltet.

**4.** Variante des Verfahrens nach Anspruch 1, dadurch gekennzeichnet, daß man zur Herstellung der β-Hydroxysäure der Formel VI von Anspruch 1:

a) ein β-Hydroxy-δ-lacton der Formel II von Anspruch 1 verestert,

b) den erhaltenen Ester der Formel

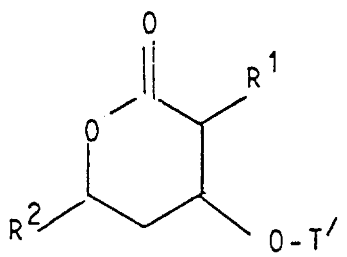

III'

worin T' Aroyl ist,
säurekatalytisch in Gegenwart eines Alkohols der Formel $R^{10}$-OH zu einem Ester der Formel

$$R^2\text{-CHOHCH}_2\text{CH(O-T')CH(R}^1\text{)COO-R}^{10} \qquad \text{IV-B}$$

öffnet,

c) den Ester der Formel IV-B veräthert und

d) den erhaltenen Aetherdiester der Formel

$$R^2\text{-CH(OCH}_2\text{-Ar)CH}_2\text{CH(O-T')CH(R}^1\text{)COO-R}^{10} \qquad \text{V-B}$$

doppelt verseift.

**5.** Verfahren nach Anspruch 1 oder 4, dadurch gekennzeichnet, daß man zur Herstellung der β-Hydroxy-δ-lactone der Formel II von Anspruch 1:

a) einen β-Hydroxyester der Formel

$$R^2\text{-CHOHCH}_2\text{COO-R} \qquad \text{VIII}$$

worin R $C_{1\text{-}4}$-Alkyl ist,
verseift,

b) das Imidazolid der erhaltenen β-Hydroxysäure der Formel

$$R^2\text{-CHOHCH}_2\text{COOH} \qquad \text{IX}$$

mit dem Mg-Salz des Malonsäureesterderivats der Formel

$$\text{HOCOCH(R}^1\text{)COO-R} \qquad \text{X}$$

umsetzt,

c) den entstandenen δ-Hydroxy-β-ketoester der Formel

$$R^2\text{-CHOHCH}_2\text{COCH(R}^1\text{)COO-R} \qquad \text{XI}$$

cyclisiert und

d) das erhaltene β-Keto-δ-lacton der Formel

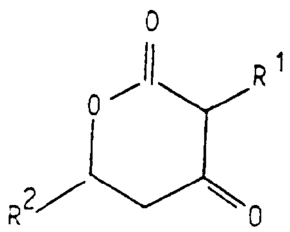

XII

katalytisch hydriert.

**6.** Verfahren nach Anspruch 1 oder 4, dadurch gekennzeichnet, daß man zur Herstellung der β-Hydroxy-δ-lactone der Formel II von Anspruch 1:

a) einen β-Ketoester der Formel

$$CH_3\text{COCH(R}^1\text{)COO-R} \qquad \text{XIII}$$

mit einem Ester der Formel

$$R^2\text{-COO-R} \qquad \text{XIV}$$

umsetzt,

b) den erhaltenen Diketoester der Formel

$$R^2\text{-}COCH_2COCH(R^1)COO\text{-}R \qquad XV$$

cyclisiert und

c) das erhaltene Pyron der Formel

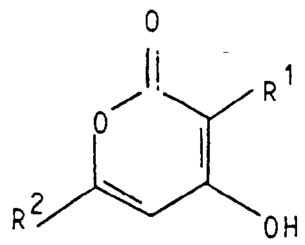

XVI

katalytisch hydriert.

**7.** Verfahren nach Anspruch 1 oder 5, dadurch gekennzeichnet, dass man zur Herstellung der Verbindung der Formel II:

a) einen β-Ketoester der Formel

$$CH_3COCH(R^1)COO\text{-}R \qquad XIII$$

mit einem Aldehyd der Formel

$$R^2\text{-}CHO \qquad XVII$$

umsetzt,

b) den entstandenen δ-Hydroxy-β-ketoester der Formel

$$R^2\text{-}CHOHCH_2COCH(R^1)COO\text{-}R \qquad XI$$

cyclisiert und

c) das erhaltene β-Keto-δ-lacton der Formel

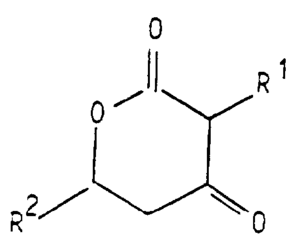

XII

katalytisch hydriert.

**8.** Variante des Verfahrens nach Anspruch 5, dadurch gekennzeichnet, daß man zur Herstellung der β-Keto-δ-lactone der Formel XII von Anspruch 5:

a) einen β-Hydroxyester der obigen Formel

$$R^2\text{-CHOHCH}_2\text{COO-R} \qquad \text{VIII}$$

veräthert,

b) den erhaltenen Aether der Formel

$$R^2\text{-CH(OCH}_2\text{-Ar)CH}_2\text{COO-R} \qquad \text{XVIII}$$

verseift,

c) die erhaltene Aethersäure der Formel

$$R^2\text{-CH(OCH}_2\text{-Ar)CH}_2\text{COOH} \qquad \text{XIX}$$

halogeniert,

d) das erhaltene Säurehalogenid mit Meldrumsäure umsetzt,

e) die erhaltene Verbindung der Formel

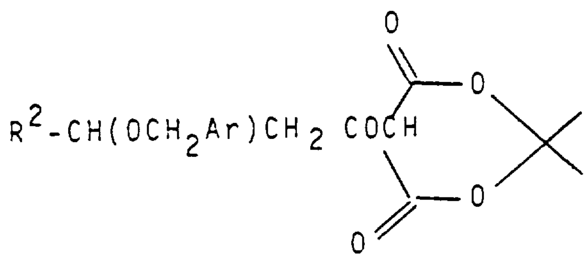

XX

hydrogenolysiert und zu einem β-Keto-δ-lacton der Formel

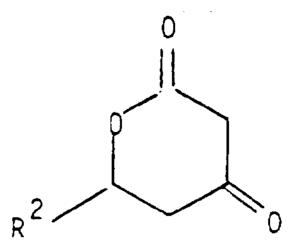

XXI

cyclisiert, und

f) das β-Keto-δ-lacton der obigen Formel XXI mit einem die Gruppe $R^1$ bzw. $-CH_2-R^{11}$ einführenden Aldehyd der Formel

$$R^{11}\text{-CHO} \qquad \text{XXII}$$

worin $R^{11}$ zusammen mit der Methylengruppe die Gruppe $R^1$ darstellt,
zu einem β-Keto-δ-lacton der obigen Formel XII umsetzt.

**9.** Variante des Verfahrens nach Anspruch 8, dadurch gekennzeichnet, daß man zur Herstellung der β-Keto-δ-lactonen der Formel XXI von Anspruch 8:

a) das Imidazolid der β-Hydroxysäure der Formel

$$R^2\text{-}CHOHCH_2COOH \qquad IX$$

mit dem Mg-Salz eines Malonsäuremononiederalkylesters umsetzt und

b) den entstandenen δ-Hydroxy-β-ketoester der Formel

$$R^2\text{-}CHOHCH_2COCH_2COO\text{-}R \qquad XXIII$$

zum β-Keto-δ-lacton der obigen Formel XXI cyclisiert.

**10.** Verfahren nach Anspruch 1, 2, 5 oder 7, worin $R^1$ n-Hexyl und $R^2$ Undecyl ist.

**Claims**

**Claims for the following Contracting States : AT, BE, CH, DE, DK, FR, GB, GR, IT, LI, LU, NL, SE**

**1.** A process for the manufacture of oxetanones of the formula

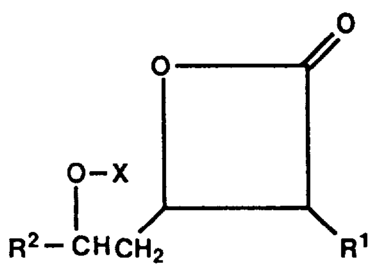

I

wherein

| | |
|---|---|
| $R^1$ and $R^2$ | are alkyl with up to 17 C atoms optionally interrupted by an O atom in a position other than the α- or β-position; or benzyl optionally ring-substituted by 1 to 3 $C_{1-6}$-alkyl or $C_{1-6}$-alkoxy groups, |
| X | is hydrogen or a group of the formula $(R^3,R^4)NCH(R^5)(CH_2)_n$-CO- |
| $R^3$ | is hydrogen, $C_{1-3}$-alkyl or $C_{1-3}$-alkanoyl, |
| $R^4$ | is hydrogen or $C_{1-3}$-alkyl and |
| $R^5$ | is hydrogen, a group Ar or Ar-$C_{1-3}$-alkyl or $C_{1-7}$-alkyl optionally interrupted by Y and optionally substituted by Z or |
| $R^4$ with $R^5$ | form together with the N atom to which they are attached a 4- to 6-membered saturated ring, |
| Y | is oxygen, sulphur or a group $N(R^6)$, $C(O)N(R^6)$ or $N(R^6)C(O)$, |
| Z | is a group -(O or S)-$R^7$, -$N(R^7,R^8)$, -$C(O)N(R^7,R^8)$ or -$N(R^7)C(O)R^8$, |
| n | is the number 1 or 0, whereby $R^5$ is hydrogen when n is the number 1, |
| Ar | is phenyl substituted by 1 to 3 groups $R^9$ or $OR^9$ and |
| $R^6$ to $R^9$ | are hydrogen or $C_{1-3}$-alkyl, |

and of salts of the oxetanones of formula I in which X is not hydrogen with weak acids, characterized by

a) etherifying a β-hydroxy-δ-lactone of the formula

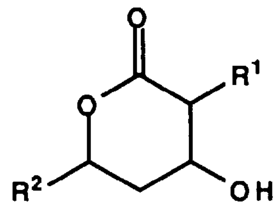

II,

b) opening the resulting ether of the formula

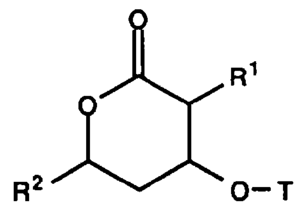

III

wherein T is a readily cleavable ether group,
with a base,

c) reacting the resulting salt of the formula

$R^2$-CHOHCH$_2$CH(O-T)CH($R^1$)COO-M    IV

wherein M is an alkali metal or alkaline earth metal,
in optional sequence with an arylmethyl halide and a base and

d) selectively cleaving the resulting diether of the formula

$R^2$-CH(OCH$_2$-Ar)CH$_2$CH(O-T)CH($R^1$)COO-M    V

with an acid,

e) cyclizing the resulting β-hydroxyacid of the formula

$R^2$-CH(OCH$_2$-Ar)CH$_2$CHOHCH($R^1$)COOH    VI,

optionally after resolution into its enantiomers,

f) cleaving the resulting β-lactone ether of the formula

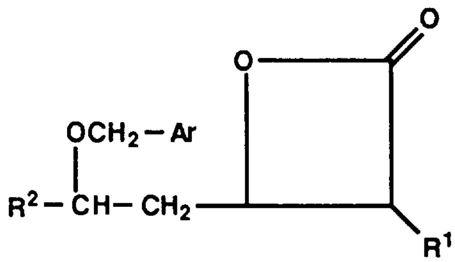

VII,

and

g) if desired, esterifying the resulting β-lactone alcohol of formula I in which X is hydrogen with an agent which introduces the group X and

h) if desired, isolating the ester obtained in the form of a salt with a weak acid.

**2.** A process according to claim 1, characterized in that an alkali metal hydride is used as the base in step c).

**3.** A variant of the process according to claim 1, characterized in that for the preparation of the β-hydroxyacid of formula VI of claim 1:

a) a salt of formula IV of claim 1 is esterified with a halide of the formula $R^{10}$-Hal, wherein $R^{10}$ is $C_{1-4}$-alkyl or aryl-$C_{1-4}$-alkyl,

b) the resulting ester of the formula

$$R^2\text{-CHOHCH}_2\text{CH(O-T)CH(R}^1\text{)COO-R}^{10} \qquad \text{IV-A}$$

is etherified and

c) the resulting diether of the formula

$$R^2\text{-CH(OCH}_2\text{-Ar)CH}_2\text{CH(O-T)CH(R}^1\text{)COO-R}^{10} \qquad \text{V-A}$$

is saponified and cleaved in the β-position in optional sequence.

**4.** A variant of the process according to claim 1, characterized in that for the preparation of the β-hydroxyacid of formula VI of claim 1:

a) a β-hydroxy-δ-lactone of formula II of claim 1 is esterified,

b) the resulting ester of the formula

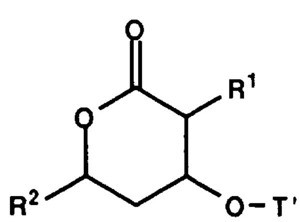

III'

wherein T' is aroyl,
is opened by acidic catalysis in the presence of an alcohol of the formula $R^{10}$-OH to give an ester of the formula

$$R^2\text{-CHOHCH}_2\text{CH(O-T')CH(R}^1\text{)COO-R}^{10} \qquad \text{IV-B,}$$

c) the ester of formula IV-B is etherified and

d) the resulting ether diester of the formula

$$R^2\text{-CH(OCH}_2\text{-Ar)CH}_2\text{CH(O-T')CH(R}^1\text{)COO-R}^{10} \qquad \text{V-B}$$

is doubly saponified.

**5.** A process according to claim 1 or 4, characterized in that for the preparation of the β-hydroxy-δ-lactones of formula II of claim 1:

a) a β-hydroxyester of the formula

$$R^2\text{-CHOHCH}_2\text{COO-R} \qquad \text{VIII}$$

wherein R is $C_{1-4}$-alkyl,
is saponified,

b) the imidazolide of the resulting β-hydroxyacid of the formula

$$R^2\text{-CHOHCH}_2\text{COOH} \qquad \text{IX}$$

is reacted with the Mg salt of the malonic acid ester derivative of the formula

$$\text{HOCOCH(R}^1\text{)COO-R} \qquad \text{X,}$$

c) the resulting δ-hydroxy-β-ketoester of the formula

$$R^2\text{-CHOHCH}_2\text{COCH(R}^1\text{)COOR} \qquad \text{XI}$$

is cyclized and

d) the resulting β-keto-δ-lactone of the formula

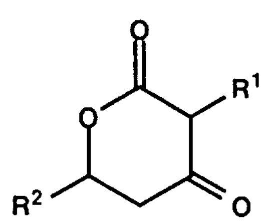

XII

is catalytically hydrogenated.

**6.** A process according to claim 1 or 4, characterized in that for the preparation of the β-hydroxy-δ-lactones of formula II of claim 1:

a) a β-ketoester of the formula

$$CH_3COCH(R^1)COO\text{-}R \qquad XIII$$

is reacted with an ester of the formula

$$R^2\text{-}COO\text{-}R \qquad XIV,$$

b) the resulting diketoester of the formula

$$R^2\text{-}COCH_2COCH(R^1)COO\text{-}R \qquad XV$$

is cyclized and

c) the resulting pyrone of the formula

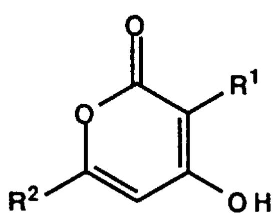

XVI

is catalytically hydrogenated.

**7.** A process according to claim 1 or 5, characterized in that for the preparation of the compound of formula II:

a) a β-ketoester of the formula

$$CH_3COCH(R^1)COO\text{-}R \qquad XIII$$

is reacted with an aldehyde of the formula

$$R^2\text{-}CHO \qquad XVII,$$

b) the resulting δ-hydroxy-β-ketoester of the formula

$$R^2\text{-}CHOHCH_2COCH(R^1)COO\text{-}R \qquad XI$$

is cyclized and

c) the resulting β-keto-δ-lactone of the formula

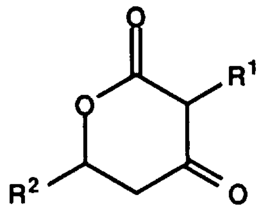

XII

is catalytically hydrogenated.

8. A variant of the process according to claim 5, characterized in that for the preparation of the β-keto-δ-lactones of formula XII of claim 5:

a) a β-hydroxyester of the above formula

$$R^2\text{-CHOHCH}_2\text{COO-R} \qquad \text{VIII}$$

is etherified,

b) the resulting ether of the formula

$$R^2\text{-CH(OCH}_2\text{-Ar)CH}_2\text{COO-R} \qquad \text{XVIII}$$

is saponified,

c) the resulting ether acid of the formula

$$R^2\text{-CH(OCH}_2\text{-Ar)CH}_2\text{COOH} \qquad \text{XIX}$$

is halogenated,

d) the resulting acid halide is reacted with Meldrum acid,

e) the resulting compound of the formula

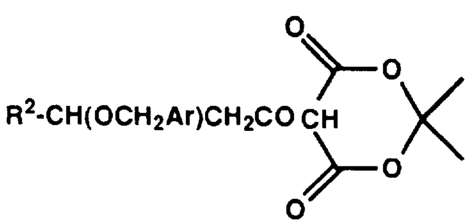

XX

is hydrogenolyzed and cyclized to a β-keto-δ-lactone of the formula

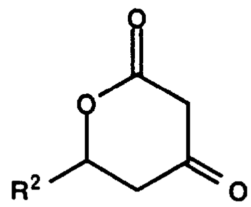

XXI

and

f) the β-keto-δ-lactone of formula XXI above is reacted with an aldehyde which introduces the group $R^1$ or $-CH_2-R^{11}$ and which has the formula

$$R^{11}\text{-CHO} \qquad \text{XXII}$$

wherein $R^{11}$ together with the methylene group represents the group $R^1$, to give a β-keto-δ-lactone of formula XII above.

**9.** A variant of the process according to claim 8, characterized in that for the preparation of the β-keto-δ-lactones of formula XXI of claim 8:

a) the imidazolide of the β-hydroxyacid of the formula

$$R^2\text{-CHOHCH}_2\text{COOH} \qquad \text{IX}$$

is reacted with the Mg salt of a mono-lower alkyl malonate and

b) the resulting δ-hydroxy-β-ketoester of the formula

$$R^2\text{-CHOHCH}_2\text{COCH}_2\text{COO-R} \qquad \text{XXIII}$$

is cyclized to the β-keto-δ-lactone of formula XXI above.

**10.** A process according to claim 1, 2, 5 or 7, wherein $R^1$ is n-hexyl and $R^2$ is undecyl.

**11.** The β-hydroxy-δ-lactones, β-keto-δ-lactones and pyrones of formulae II, XII and, respectively, XVI

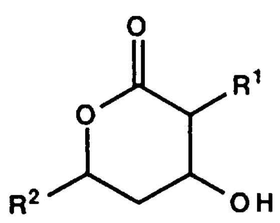

,

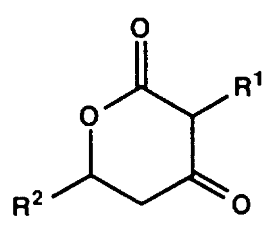

,

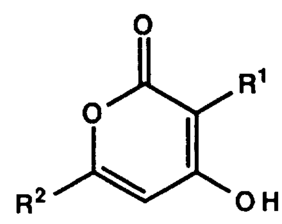

II XII XVI

from the following group:

rac-(2RS,3RS,5SR)-2-hexyl-3-hydroxy-5-undecyl-δ-valeriolactone,

rac-(2RS,3RS,5SR)-2-ethyl-5-heptadecyl-3-hydroxy-δ-valeriolactone,
(2S,3S,5R)-2-ethyl-5-heptadecyl-3-hydroxy-δ-valeriolactone and
rac-(2RS,3RS,5SR)-2-hexyl-3-hydroxy-5-pentyl-δ-valeriolactone;
rac-5,6-dihydro-3-hexyl-4-hydroxy-6-undecyl-2H-pyran-2-one,
(R)-3-ethyl-5,6-dihydro-6-heptadecyl-4-hydroxy-2H-pyran-2-one and
rac-5,6-dihydro-3-hexyl-4-hydroxy-6-pentyl-2H-pyran-2-one;
3-hexyl-4-hydroxy-6-undecyl-2H-pyran-2-one,
3-ethyl-6-heptadecyl-4-hydroxy-2H-pyran-2-one and
3-hexyl-4-hydroxy-6-pentyl-2H-pyran-2-one.

**12.** The β-keto-δ-lactones of formula XXI of claim 8 in which $R^2$ has the significance given in claim 1, but the number of C atoms in the alkyl group $R^2$ amounts to more than 9, especially the following:

(R)-5,6-dihydro-6-undecyl-2H-pyran-2,4(3H)-dione and
(R)-5,6-dihydro-6-heptdecyl-2H-pyran-2,4(3H)-dione.

**Claims for the following Contracting State : ES**

**1.** A process for the manufacture of oxetanones of the formula

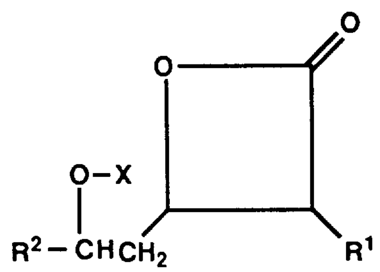

I

wherein

| | |
|---|---|
| $R^1$ and $R^2$ | are alkyl with up to 17 C atoms optionally interrupted by an O atom in a position other than the α- or β-position; or benzyl optionally ring-substituted by 1 to 3 $C_{1-6}$-alkyl or $C_{1-6}$-alkoxy groups, |
| X | is hydrogen or a group of the formula $(R^3,R^4)NCH(R^5)(CH_2)_n$-CO- |
| $R^3$ | is hydrogen, $C_{1-3}$-alkyl or $C_{1-3}$-alkanoyl, |
| $R^4$ | is hydrogen or $C_{1-3}$-alkyl and |
| $R^5$ | is hydrogen, a group Ar or Ar-$C_{1-3}$-alkyl or $C_{1-7}$-alkyl optionally interrupted by Y and optionally substituted by Z or |
| $R^4$ with $R^5$ | form together with the N atom to which they are attached a 4- to 6-membered saturated ring, |
| Y | is oxygen, sulphur or a group $N(R^6)$, $C(O)N(R^6)$ or $N(R^6)C(O)$, |
| Z | is a group -(O or S)-$R^7$, $-N(R^7,R^8)$, $-C(O)N(R^7,R^8)$ or $-N(R^7)C(O)R^8$, |
| n | is the number 1 or 0, whereby $R^5$ is hydrogen when n is the number 1, |
| Ar | is phenyl substituted by 1 to 3 groups $R^9$ or $OR^9$ and |
| $R^6$ to $R^9$ | are hydrogen or $C_{1-3}$-alkyl, |

and of salts of the oxetanones of formula I in which X is not hydrogen with weak acids, characterized by

a) etherifying a β-hydroxy-δ-lactone of the formula

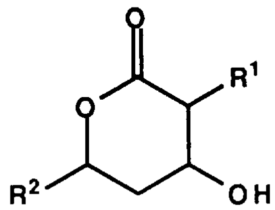

II,

b) opening the resulting ether of the formula

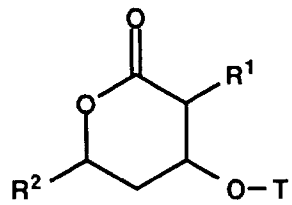

III

wherein T is a readily cleavable ether group,
with a base,

c) reacting the resulting salt of the formula

$$R^2\text{-CHOHCH}_2\text{CH(O-T)CH(R}^1\text{)COO-M} \quad \text{IV}$$

wherein M is an alkali metal or alkaline earth metal,
in optional sequence with an arylmethyl halide and a base and

d) selectively cleaving the resulting diether of the formula

$$R^2\text{-CH(COH}_2\text{-Ar)CH}_2\text{CH(O-T)CH(R}^1\text{)COO-M} \quad \text{V}$$

with an acid,

e) cyclizing the resulting β-hydroxyacid of the formula

$$R^2\text{-CH(OCH}_2\text{-Ar)CH}_2\text{CHOHCH(R}^1\text{)COOH} \quad \text{VI,}$$

optionally after resolution into its enantiomers,

f) cleaving the resulting β-lactone ether of the formula

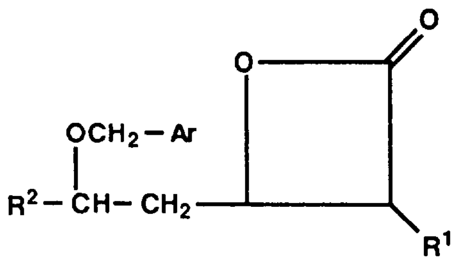

VII,

and

g) if desired, esterifying the resulting β-lactone alcohol of formula I in which X is hydrogen with an agent which introduces the group X and

h) if desired, isolating the ester obtained in the form of a salt with a weak acid.

**2.** A process according to claim 1, characterized in that an alkali metal hydride is used as the base in step c).

**3.** A variant of the process according to claim 1, characterized in that for the preparation of the β-hydroxyacid of formula VI of claim 1:

a) a salt of formula IV of claim 1 is esterified with a halide of the formula $R^{10}$-Hal, wherein $R^{10}$ is $C_{1-4}$-alkyl or aryl-$C_{1-4}$-alkyl,

b) the resulting ester of the formula

$$R^2\text{-CHOHCH}_2\text{CH(O-T)CH(}R^1\text{)COO-}R^{10} \qquad \text{IV-A}$$

is etherified and

c) the resulting diether of the formula

$$R^2\text{-CH(OCH}_2\text{-Ar)CH}_2\text{CH(O-T)CH(}R^1\text{)COO-}R^{10} \qquad \text{V-A}$$

is saponified and cleaved in the β-position in optional sequence.

**4.** A variant of the process according to claim 1, characterized in that for the preparation of the β-hydroxyacid of formula VI of claim 1:

a) a β-hydroxy-δ-lactone of formula II of claim 1 is esterified,

b) the resulting ester of the formula

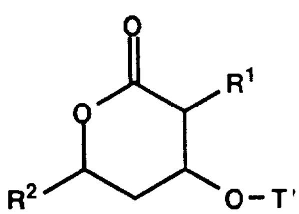

III'

wherein T' is aroyl,

is opened by acidic catalysis in the presence of an alcohol of the formula $R^{10}$-OH to give an ester of the formula

$$R^2\text{-CHOHCH}_2\text{CH(O-T')CH(}R^1\text{)COO-}R^{10} \qquad \text{IV-B,}$$

c) the ester of formula IV-B is etherified and

d) the resulting ether diester of the formula

$$R^2\text{-CH(OCH}_2\text{-Ar)CH}_2\text{CH(O-T')CH(}R^1\text{)COO-}R^{10} \qquad \text{V-B}$$

is doubly saponified.

**5.** A process according to claim 1 or 4, characterized in that for the preparation of the β-hydroxy-δ-lactones of formula II of claim 1:

a) a β-hydroxyester of the formula

$$R^2\text{-CHOHCH}_2\text{COO-R} \qquad \text{VIII}$$

wherein R is $C_{1-4}$-alkyl,
is saponified,

b) the imidazolide of the resulting β-hydroxyacid of the formula

$$R^2\text{-CHOHCH}_2\text{COOH} \qquad \text{IX}$$

is reacted with the Mg salt of the malonic acid ester derivative of the formula

$$\text{HOCOCH(}R^1\text{)COO-R} \qquad \text{X,}$$

c) the resulting δ-hydroxy-β-ketoester of the formula

$$R^2\text{-CHOHCH}_2\text{COCH(}R^1\text{)COO-R} \qquad \text{XI}$$

is cyclized and

d) the resulting β-keto-δ-lactone of the formula

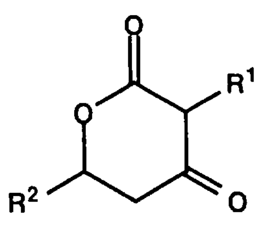

XII

is catalytically hydrogenated.

**6.** A process according to claim 1 or 4, characterized in that for the preparation of the β-hydroxy-δ-lactones of formula

II of claim 1:

a) a β-ketoester of the formula

$$CH_3COCH(R^1)COO\text{-}R \qquad XIII$$

is reacted with an ester of the formula

$$R^2\text{-}COO\text{-}R \qquad XIV,$$

b) the resulting diketoester of the formula

$$R^2\text{-}COCH_2COCH(R^1)COO\text{-}R \qquad XV$$

is cyclized and

c) the resulting pyrone of the formula

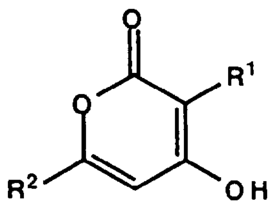

XVI

is catalytically hydrogenated.

**7.** A process according to claim 1 or 5, characterized in that for the preparation of the compound of formula II:

a) a β-ketoester of the formula

$$CH_3COCH(R^1)COO\text{-}R \qquad XIII$$

is reacted with an aldehyde of the formula

$$R^2\text{-}CHO \qquad XVII,$$

b) the resulting δ-hydroxy-β-ketoester of the formula

$$R^2\text{-}CHOHCH_2COCH(R^1)COO\text{-}R \qquad XI$$

is cyclized and

c) the resulting β-keto-δ-lactone of the formula

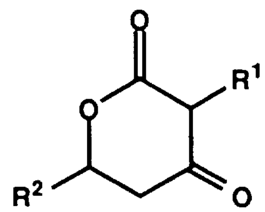

XII

is catalytically hydrogenated.

**8.** A variant of the process according to claim 5, characterized in that for the preparation of the β-keto-δ-lactones of formula XII of claim 5:

a) a β-hydroxyester of the above formula

$$R^2\text{-CHOHCH}_2\text{COO-R} \qquad \text{VIII}$$

is etherified,

b) the resulting ether of the formula

$$R^2\text{-CH(OCH}_2\text{-Ar)CH}_2\text{COO-R} \qquad \text{XVIII}$$

is saponified,

c) the resulting ether acid of the formula

$$R^2\text{-CH(OCH}_2\text{-Ar)CH}_2\text{COOH} \qquad \text{XIX}$$

is halogenated,

d) the resulting acid halide is reacted with Meldrum acid,

e) the resulting compound of the formula

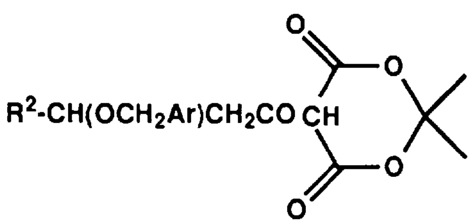

XX

is hydrogenolyzed and cyclized to a β-keto-δ-lactone of the formula

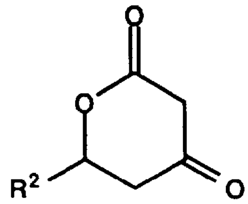

XXI

and

f) the β-keto-δ-lactone of formula XXI above is reacted with an aldehyde which introduces the group $R^1$ or $-CH_2-R^{11}$ and which has the formula

$$R^{11}\text{-CHO} \qquad \text{XXII}$$

wherein $R^{11}$ together with the methylene group represents the group $R^1$,
to give a β-keto-δ-lactone of formula XII above.

**9.** A variant of the process according to claim 8, characterized in that for the preparation of the β-keto-δ-lactones of formula XXI of claim 8:

a) the imidazolide of the β-hydroxyacid of the formula

$$R^2\text{-CHOHCH}_2\text{COOH} \qquad \text{IX}$$

is reacted with the Mg salt of a mono-lower alkyl malonate and

b) the resulting δ-hydroxy-β-ketoester of the formula

$$R^2\text{-CHOHCH}_2\text{COCH}_2\text{COO-R} \qquad \text{XXIII}$$

is cyclized to the β-keto-δ-lactone of formula XXI above.

**10.** A process according to claim 1, 2, 5 or 7, wherein $R^1$ is n-hexyl and $R^2$ is undecyl.

**Revendications**

**Revendications pour les Etats contractants suivants : AT, BE, CH, DE, DK, FR, GB, IT, LI, LU, NL, SE, GR**

**1.** Procédé de préparation d'oxétanones de formule

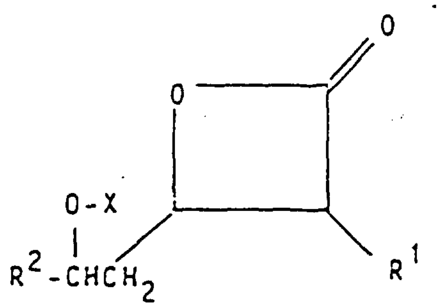

I

dans laquelle

$R^1$ et $R^2$ représentent un alkyle ayant jusqu'à 17 atomes de carbone éventuellement interrompu en une position autre que α ou β par un atome d'oxygène; ou un benzyle éventuellement substitué dans son noyau par 3 groupes alkyle en $C_1$ à $C_6$ ou alcoxy en $C_1$ à $C_6$,
X représente un hydrogène ou un groupe de formule

$$(R^3,R^4)NCH(R^5)(CH_2)_n\text{-CO-}$$

$R^3$ représente un hydrogène, un alkyle en $C_1$ à $C_3$ ou un alcanoyle en $C_1$ à $C_3$,
$R^4$ représente un hydrogène ou un alkyle en $C_1$ à $C_3$ , et
$R^5$ représente un hydrogène, un groupe Ar ou Ar-alkyle en $C_1$ à $C_3$ ou un alkyle en $C_1$ à $C_7$ éventuellement interrompu par Y et éventuellement substitué par Z, ou $R^4$ et $R^5$ forment ensemble avec l'atome d'azote auquel ils sont liés un noyau saturé à 4 à 6 chaînons,
Y représente un oxygène, un soufre ou un groupe $N(R^6)$, $C(O)N(R^6)$ ou $N(R^6)C(O)$,
Z représente un groupe -(O ou S)-$R^7$, -$N(R^7,R^8)$, -$C(O)N(R^7,R^8)$ ou -$N(R^7)C(O)R^8$,
n est le nombre 1 ou 0, et si n est le nombre 1, $R^5$ est un hydrogène,
Ar est un phényle substitué par de 1 à 3 groupes $R^9$ ou $OR^9$, et
$R^6$ à $R^9$ représentent un hydrogène ou un alkyle en $C_1$ à $C_3$,

et de sels des oxétanones de formule I, dans laquelle X n'est pas un hydrogène, avec des acides faibles, caractérisé en ce que

a) on éthérifie une β-hydroxy-δ-lactone de formule

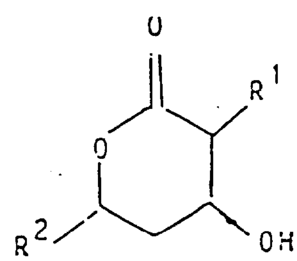

II

b) on ouvre avec une base l'éther obtenu de formule

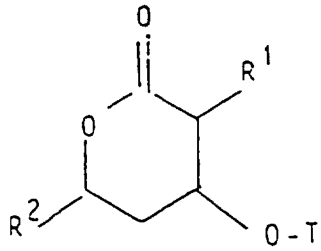

III

dans laquelle T est un groupe éther facilement séparable,
c) on fait réagir le sel obtenu de formule

$$R^2\text{-CHOHCH}_2\text{CH(O-T)CH(R}^1\text{)COO-M} \quad \text{IV}$$

dans laquelle M est un sel de métal alcalin ou alcalino-terreux,
dans un ordre quelconque avec un halogénure d'arylméthyle et une base et
d) on clive de façon sélective avec un acide le diéther apparu de formule

$$R^2\text{-CH(OCH}_2\text{-Ar)CH}_2\text{CH(O-T)CH(R}^1\text{)COO-M} \quad \text{V}$$

e) on cyclise le β-hydroxyacide obtenu de formule

$$R^2\text{-CH(OCH}_2\text{-Ar)CH}_2\text{CHOHCH(R}^1\text{)COOH} \quad \text{VI}$$

le cas échéant après clivage pour obtenir ses énantiomères,
f) on clive l'éther de β-lactone obtenu de formule

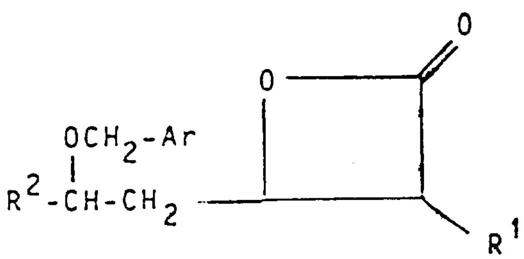

VII

et
g) on estérifie l'alcool de β-lactone obtenu de formule I, dans laquelle X est un hydrogène, le cas échéant avec un agent introduisant le groupe X et
h) on isole l'ester obtenu, le cas échéant sous la forme d'un sel avec un acide faible.

**2.** Procédé selon la revendication 1, caractérisé en ce qu'on utilise comme base dans l'étape c) un hydrure de métal alcalin.

**3.** Variante du procédé selon la revendication 1, caractérisée en ce que pour préparer le β-hydroxyacide de formule VI de la revendication 1:

a) on estérifie un sel de formule IV de la revendication 1 avec un halogénure de formule $R^{10}$-Hal, dans laquelle $R^{10}$ est un alkyle en $C_1$ à $C_4$ ou un arylalkyle en $C_1$ à $C_4$ ,
b) on éthérifie l'ester obtenu de formule

$$R^2\text{-CHOHCH}_2\text{CH(O-T)CH}(R^1)\text{COO-}R^{10} \qquad \text{IV-A}$$

et
c) on saponifie le diéther obtenu de formule

$$R^2\text{-CH(OCH}_2\text{-Ar)CH}_2\text{CH(OT)CH}(R^1)\text{COO-}R^{10} \qquad \text{V-A}$$

et on le clive en position β , dans un ordre quelconque.

**4.** Variante du procédé selon la revendication 1, caractérisée en ce que pour préparer le β-hydroxyacide de formule VI de la revendication 1:

a) on estérifie une β-hydroxy-δ-lactone de formule II de la revendication 1,
b) on ouvre l'ester obtenu de formule

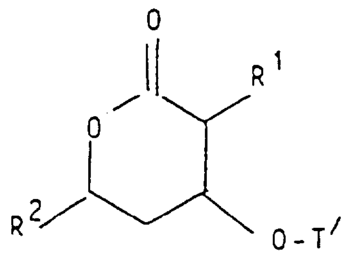

III'

dans laquelle T' est un aroyle,
par catalyse acide en présence d'un alcool de formule $R^{10}$-OH pour donner un ester de formule

$$R^2\text{-CHOHCH}_2\text{CH(O-T')CH}(R^1)\text{COO-}R^{10} \qquad \text{IV-B}$$

c) on éthérifie l'ester de formule IV-B et
d) on saponifie doublement l'éther-diester obtenu de formule

$$R^2\text{-CH(OCH}_2\text{-Ar)CH}_2\text{CH(O-T')CH}(R^1)\text{COO-}R^{10} \qquad \text{V-B}$$

**5.** Procédé selon la revendication 1 ou 4, caractérisé en ce que pour préparer les β-hydroxy-δ-lactones de formule III de la revendication 1:

a) on saponifie un β-hydroxyester de formule

$$R^2\text{-CHOHCH}_2\text{COO-R} \qquad \text{VIII}$$

dans laquelle R est un alkyle en $C_1$ à $C_4$ ,
b) on fait réagir l'imidazolide du β-hydroxy acide obtenu de formule

$$R^2\text{-CHOHCH}_2\text{COOH} \qquad \text{IX}$$

avec le sel de magnésium du dérivé ester d'acide malonique de formule

$HOCOCH(R^1)COO\text{-}R$ X

c) on cyclise le δ-hydroxy-β-cétoester apparu de formule

$R^2\text{-}CHOHCH_2COCH(R^1)COO\text{-}R$ XI

et
d) on hydrogène de façon catalytique la β-céto-δ-lactone obtenue de formule

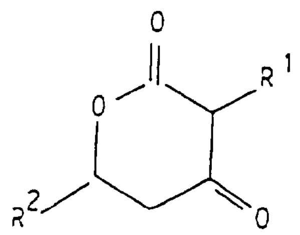

XII

**6.** Procédé selon la revendication 1 ou 4, caractérisé en ce que pour préparer les β-hydroxy-δ-lactones de formule II de la revendication 1:

a) on fait réagir un β-cétoester de formule

$CH_3COCH(R^1)COO\text{-}R$ XIII

avec un ester de formule

$R^2\text{-}COO\text{-}R$ XIV

b) on cyclise le dicétoester obtenu de formule

$R^2\text{-}COCH_2COCH(R^1)COO\text{-}R$ XV

et
c) on hydrogène de façon catalytique la pyrone obtenue de formule

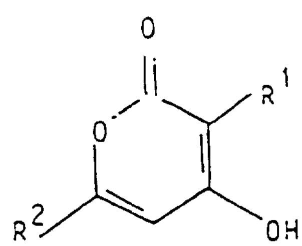

XVI

**7.** Procédé selon la revendication 1 ou 5, caractérisé en ce que pour préparer le composé de formule II:

a) on fait réagir un β-cétoester de formule

$CH_3COCH(R^1)COO\text{-}R$ XIII

avec un aldéhyde de formule

$R^2\text{-}CHO$ XVII

b) on cyclise le δ-hydroxy-β-cétoester apparu de formule

$R^2\text{-}CHOHCH_2COCH(R^1)COO\text{-}R$ XI

et

c) on hydrogène de façon catalytique la β-céto-δ-lactone obtenue de formule

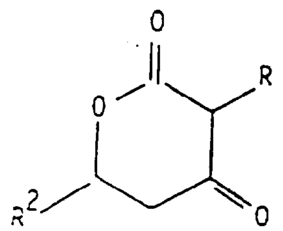

XII

**8.** Variante du procédé selon la revendication 5, caractérisée en ce que pour préparer les β-céto-δ-lactones de formule XII de la revendication 5:

a) on éthérifie un β-hydroxyester de formule ci-dessus

$R^2\text{-}CHCOCH_2COO\text{-}R$ VIII

b) on saponifie l'éther obtenu de formule

$R^2\text{-}CH(OCH_2\text{-}Ar)CH_2COO\text{-}R$ XVIII

c) on halogène l'éther-acide obtenu de formule

$R^2\text{-}CH(OCH_2\text{-}Ar)CH_2COOH$ XIX

d) on fait réagir l'halogénure d'acide obtenu avec de l'acide de Meldrum, .

e) on hydrogénolyse le composé obtenu de formule

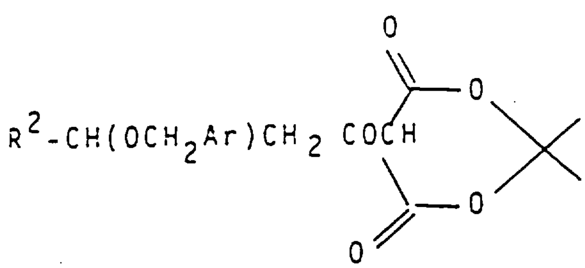

XX

et on le cyclise en une β-céto-δ-lactone de formule

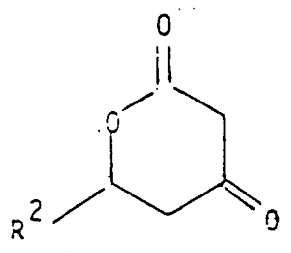

XXI

et

f) on fait réagir la β-céto-δ-lactone de formule XXI ci-dessus avec un aldéhyde introduisant le groupe $R^1$ ou selon les cas $-CH_2-R^{11}$ , de formule

$$R^{11}\text{-CHO} \qquad \text{XXII}$$

dans laquelle $R^{11}$ représente avec le groupe méthylène le groupe $R^1$ ,

pour donner une β-céto-δ-lactone de formule XII ci-dessus.

**9.** Variante du procédé selon la revendication 8, caractérisée en ce que pour préparer les β-céto-δ-lactones de formule XXI de la revendication 8:

a) on fait réagir l'imidazolide du β-hydroxy-acide de formule

$$R^2\text{-CHOHCH}_2\text{COOH} \qquad \text{IX}$$

avec le sel de magnésium d'un monoester d'alkyle inférieur de l'acide malonique et

b) on cyclise le δ-hydroxy-β-cétoester apparu de formule

$$R^2\text{-CHOHCH}_2\text{COCH}_2\text{COO-R} \qquad \text{XXIII}$$

pour donner la β-céto-δ-lactone de formule XXI ci-dessus.

**10.** Procédé selon la revendication 1, 2, 5 ou 7 dans lequel $R^1$ est un n-hexyle et $R^2$ est un undécyle.

**11.** Les β-hydroxy-δ-lactones, β-céto-δ-lactones et pyrones de formules II, XII ou XVI

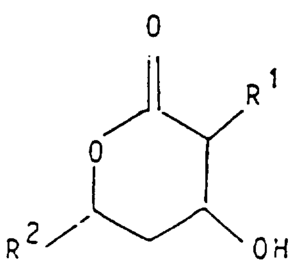 , 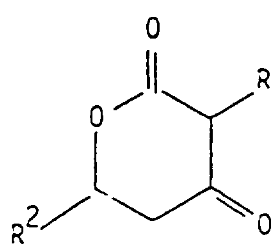 , 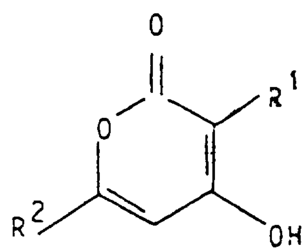

du groupe des composés suivants:

rac-(2RS,3RS,5SR)-2-hexyl-3-hydroxy-5-undécyl-δ-valériolactone,

rac-(2RS,3RS,5SR)-2-éthyl-5-heptadécyl-3-hydroxy-δ-valériolactone,
(2S,3S,5R)-2-éthyl-5-heptadécyl-3-hydroxy-δ-valériolactone et
rac-(2RS,3RS,5SR)-2-hexyl-3-hydroxy-5-pentyl-δ-valériolactone;
rac-5,6-dihydro-3-hexyl-4-hydroxy-6-undécyl-2H-pyran-2-one,
(R)-3-éthyl-5,6-dihydro-6-heptadécyl-4-hydroxy-2H-pyran-2-one et
rac-5,6-dihydro-3-hexyl-4-hydroxy-6-pentyl-2H-pyran-2-one;
3-hexyl-4-hydroxy-6-undécyl-2H-pyran-2-one,
3-éthyl-6-heptadécyl-4-hydroxy-2H-pyran-2-one et
3-hexyl-4-hydroxy-6-pentyl-2H-pyran-2-one.

**12.** Les β-céto-δ-lactones de formule XXI de la revendication 8, dans laquelle $R^2$ a la signification donnée dans la revendication 1,
mais le nombre d'atomes de carbone dans le groupe alkyle $R^2$ doit être supérieur à 9, en particulier les suivantes:

(R)-5,6-dihydro-6-undécyl-2H-pyranne-2,4(3H)-dione et
(R)-5,6-dihydro-6-heptadécyl-2H-pyranne-2,4(3H)-dione.

**Revendications pour l'Etat contractant suivant : ES**

**1.** Procédé de préparation d'oxétanones de formule

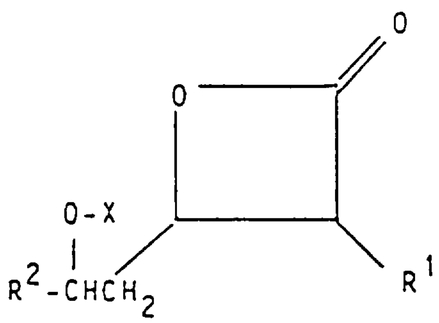

I

dans laquelle

$R^1$ et $R^2$ représentent un alkyle ayant jusqu'à 17 atomes de carbone éventuellement interrompu en une position autre que α ou β par un atome d'oxygène; ou un benzyle éventuellement substitué dans son noyau par 3 groupes alkyle en $C_1$ à $C_6$ ou alcoxy en $C_1$ à $C_6$,
X représente un hydrogène ou un groupe de formule

$$(R^3,R^4)NCH(R^5)(CH_2)_n\text{-CO-}$$

$R^3$ représente un hydrogène, un alkyle en $C_1$ à $C_3$ ou un alcanoyle en $C_1$ à $C_3$,
$R^4$ représente un hydrogène ou un alkyle en $C_1$ à $C_3$ , et
$R^5$ représente un hydrogène, un groupe Ar ou Ar-alkyle en $C_1$ à $C_3$ ou un alkyle en $C_1$ à $C_7$ éventuellement interrompu par Y et éventuellement substitué par Z, ou $R^4$ et $R^5$ forment ensemble avec l'atome d'azote auquel ils sont liés un noyau saturé à 4 à 6 chaînons,
Y représente un oxygène, un soufre ou un groupe $N(R^6)$, $C(O)N(R^6)$ ou $N(R^6)C(O)$,
Z représente un groupe -(O ou S)-$R^7$, $-N(R^7,R^8)$, $-C(O)N(R^7,R^8)$ ou $-N(R^7)C(O)R^8$,
n est le nombre 1 ou 0, et si n est le nombre 1, $R^5$ est un hydrogène,
Ar est un phényle substitué par de 1 à 3 groupes $R^9$ ou $OR^9$, et
$R^6$ à $R^9$ représentent un hydrogène ou un alkyle en $C_1$ à $C_3$,

et de sels des oxétanones de formule I, dans laquelle X n'est pas un hydrogène, avec des acides faibles, caractérisé en ce que

a) on éthérifie une β-hydroxy-δ-lactone de formule

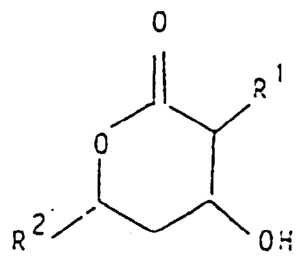

II

b) on ouvre avec une base l'éther obtenu de formule

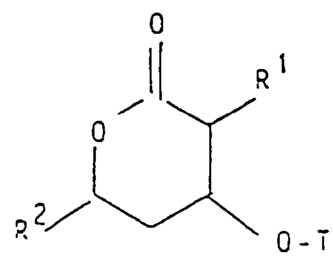

III

dans laquelle T est un groupe éther facilement séparable,
c) on fait réagir le sel obtenu de formule

$$R^2\text{-CHOHCH}_2\text{CH(O-T)CH(R}^1\text{)COO-M} \qquad \text{IV}$$

dans laquelle M est un sel de métal alcalin ou alcalino-terreux,
dans un ordre quelconque avec un halogénure d'arylméthyle et une base et
d) on clive de façon sélective avec un acide le diéther apparu de formule

$$R^2\text{-CH(OCH}_2\text{-Ar)CH}_2\text{CH(O-T)CH(R}^1\text{)COO-M} \qquad \text{V}$$

e) on cyclise le β-hydroxyacide obtenu de formule

$$R^2\text{-CH(OCH}_2\text{-Ar)CH}_2\text{CHOHCH(R}^1\text{)COOH} \qquad \text{VI}$$

le cas échéant après clivage pour obtenir ses énantiomères,
f) on clive l'éther de β-lactone obtenu de formule

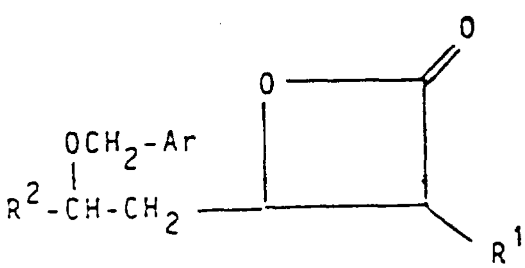

VII

et

g) on estérifie l'alcool de β-lactone obtenu de formule I, dans laquelle X est un hydrogène, le cas échéant avec un agent introduisant le groupe X et

h) on isole l'ester obtenu, le cas échéant sous la forme d'un sel avec un acide faible.

**2.** Procédé selon la revendication 1, caractérisé en ce qu'on utilise comme base dans l'étape c) un hydrure de métal alcalin.

**3.** Variante du procédé selon la revendication 1, caractérisée en ce que pour préparer le β-hydroxyacide de formule VI de la revendication 1:

a) on estérifie un sel de formule IV de la revendication 1 avec un halogénure de formule $R^{10}$-Hal, dans laquelle $R^{10}$ est un alkyle en $C_1$ à $C_4$ ou un arylalkyle en $C_1$ à $C_4$ ,

b) on éthérifie l'ester obtenu de formule

$$R^2\text{-CHOHCH}_2\text{CH(O-T)CH(R}^1\text{)COO-R}^{10} \qquad \text{IV-A}$$

et

c) on saponifie le diéther obtenu de formule

$$R^2\text{-CH(OCH}_2\text{-Ar)CH}_2\text{CH(OT)CH(R}^1\text{)COO-R}^{10} \qquad \text{V-A}$$

et on le clive en position β , dans un ordre quelconque.

**4.** Variante du procédé selon la revendication 1, caractérisée en ce que pour préparer le β-hydroxyacide de formule VI de la revendication 1:

a) on estérifie une β-hydroxy-δ-lactone de formule II de la revendication 1,

b) on ouvre l'ester obtenu de formule

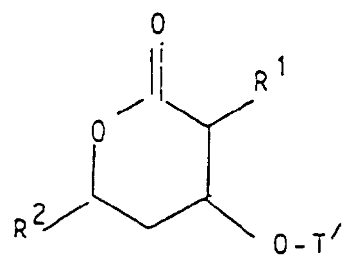

III'

dans laquelle T' est un aroyle,

par catalyse acide en présence d'un alcool de formule $R^{10}$-OH pour donner un ester de formule

$$R^2\text{-CHOHCH}_2\text{CH(O-T')CH(R}^1\text{)COO-R}^{10} \qquad \text{IV-B}$$

c) on éthérifie l'ester de formule IV-B et

d) on saponifie doublement l'éther-diester obtenu de formule

$$R^2\text{-CH(OCH}_2\text{-Ar)CH}_2\text{CH(O-T')CH(R}^1\text{)COO-R}^{10} \qquad \text{V-B}$$

**5.** Procédé selon la revendication 1 ou 4, caractérisé en ce que pour préparer les β-hydroxy-δ-lactones de formule III de la revendication 1:

a) on saponifie un β-hydroxyester de formule

$$R^2\text{-CHOHCH}_2\text{COO-R} \qquad \text{VIII}$$

dans laquelle R est un alkyle en $C_1$ à $C_4$ ,
b) on fait réagir l'imidazolide du β-hydroxy acide obtenu de formule

$$R^2\text{-CHOHCH}_2\text{COOH} \qquad \text{IX}$$

avec le sel de magnésium du dérivé ester d'acide malonique de formule

$$\text{HOCOCH}(R^1)\text{COO-R} \qquad \text{X}$$

c) on cyclise le δ-hydroxy-β-cétoester apparu de formule

$$R^2\text{-CHOHCH}_2\text{COCH}(R^1)\text{COO-R} \qquad \text{XI}$$

et
d) on hydrogène de façon catalytique la β-céto-δ-lactone obtenue de formule

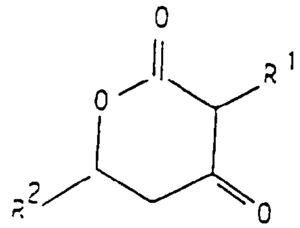

XII

**6.** Procédé selon la revendication 1 ou 4, caractérisé en ce que pour préparer les β-hydroxy-δ-lactones de formule II de la revendication 1:

a) on fait réagir un β-cétoester de formule

$$CH_3\text{COCH}(R^1)\text{COO-R} \qquad \text{XIII}$$

avec un ester de formule

$$R^2\text{-COO-R} \qquad \text{XIV}$$

b) on cyclise le dicétoester obtenu de formule

$$R^2\text{-COCH}_2\text{COCH}(R^1)\text{COO-R} \qquad \text{XV}$$

et
c) on hydrogène de façon catalytique la pyrone obtenue de formule

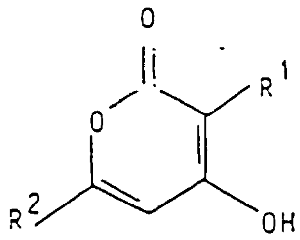

XVI

**7.** Procédé selon la revendication 1 ou 5, caractérisé en ce que pour préparer le composé de formule II:

a) on fait réagir un β-cétoester de formule

$$CH_3COCH(R^1)COO\text{-}R \qquad XIII$$

avec un aldéhyde de formule

$$R^2\text{-}CHO \qquad XVII$$

b) on cyclise le δ-hydroxy-β-cétoester apparu de formule

$$R^2\text{-}CHOHCH_2COCH(R^1)COO\text{-}R \qquad XI$$

et

c) on hydrogène de façon catalytique la β-céto-δ-lactone obtenue de formule

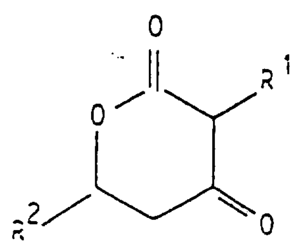

XII

**8.** Variante du procédé selon la revendication 5, caractérisée en ce que pour préparer les β-céto-δ-lactones de formule XII de la revendication 5:

a) on éthérifie un β-hydroxyester de formule ci-dessus

$$R^2\text{-}CHCOCH_2COO\text{-}R \qquad VIII$$

b) on saponifie l'éther obtenu de formule

$$R^2\text{-}CH(OCH_2\text{-}Ar)CH_2COO\text{-}R \qquad XVIII$$

c) on halogène l'éther-acide obtenu de formule

$$R^2\text{-}CH(OCH_2\text{-}Ar\ )CH_2COOH \qquad XIX$$

d) on fait réagir l'halogénure d'acide obtenu avec de l'acide de Meldrum,
e) on hydrogénolyse le composé obtenu de formule

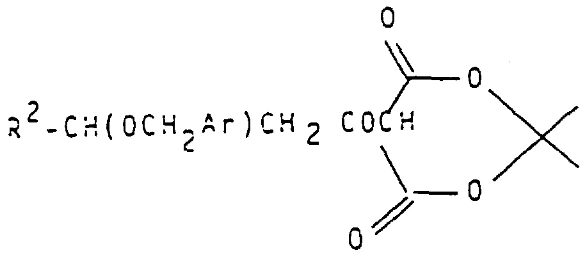

XX

et on le cyclise en une β-céto-δ-lactone de formule

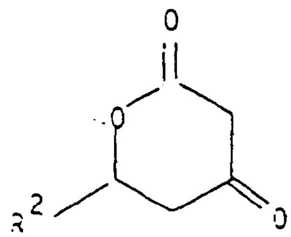

XXI

et
f) on fait réagir la β-céto-δ-lactone de formule XXI ci-dessus avec un aldéhyde introduisant le groupe $R^1$ ou selon les cas $-CH_2-R^{11}$ , de formule

$$R^{11}\text{-CHO} \qquad \text{XXII}$$

dans laquelle $R^{11}$ représente avec le groupe méthylène le groupe $R^1$ ,

pour donner une β-céto-δ-lactone de formule XII ci-dessus.

**9.** Variante du procédé selon la revendication 8, caractérisée en ce que pour préparer les β-céto-δ-lactones de formule XXI de la revendication 8:

a) on fait réagir l'imidazolide du β-hydroxy-acide de formule

$$R^2\text{-CHOHCH}_2\text{COOH} \qquad \text{IX}$$

avec le sel de magnésium d'un monoester d'alkyle inférieur de l'acide malonique et
b) on cyclise le δ-hydroxy-β-cétoester apparu de formule

$$R^2\text{-CHOHCH}_2\text{COCH}_2\text{COO-R} \qquad \text{XXIII}$$

pour donner la β-céto-δ-lactone de formule XXI ci-dessus.

**10.** Procédé selon la revendication 1, 2, 5 ou 7 dans lequel $R^1$ est un n-hexyle et $R^2$ est un undécyle.